(19)

Europäisches
Patentamt
European
Patent Office
Office européen
des brevets

(11)  **EP 1 782 773 A1**

(12)  **EUROPEAN PATENT APPLICATION**
published in accordance with Art. 158(3) EPC

(43) Date of publication:
**09.05.2007   Bulletin 2007/19**

(21) Application number: **05765766.0**

(22) Date of filing: **14.07.2005**

(51) Int Cl.:
*A61F 7/08* (2006.01)          *C09K 5/16* (2006.01)

(86) International application number:
**PCT/JP2005/013002**

(87) International publication number:
**WO 2006/006649 (19.01.2006 Gazette 2006/03)**

(84) Designated Contracting States:
**DE FR GB**

(30) Priority: **14.07.2004   JP 2004207830**

(71) Applicant: **Mycoal Products Corporation**
**Tochigi-chi, Tochigi 328-0067 (JP)**

(72) Inventor: **DODO, Toshihiro; c/o MYCOAL**
**PRODUCTS CORPORATION,**
**Tochigi 3280067 (JP)**

(74) Representative: **Thun, Clemens**
**Mitscherlich & Partner**
**Sonnenstrasse 33**
**80331 München (DE)**

(54)  **COMPRESSED WET EXOTHERMAL COMPOSITION, EXOTHERMAL ARTICLE AND METHOD FOR PRODUCING COMPRESSED WET EXOTHERMAL COMPOSITION**

(57)      An object of the invention is to provide a wettable heat generating composition compressed body which has various shapes and various sizes, is wettable and is capable of generating heat upon contact with air. Another object of the invention is to provide a heat generating body having a structure in which the foregoing wettable heat generating composition compressed body is interposed between a substrate and a covering material and at least the periphery of the wettable heat generating composition compressed body is sealed and having an exothermic part having an air-permeable part.

A wettable heat generating composition compressed body resulting from compression of a moldable heat generating composition capable of causing an exothermic reaction upon contact with air, which is **characterized in that** the heat generating composition is one prepared by subjecting a reaction mixture containing, as essential components, an iron powder, a carbon component, a reaction accelerator, a crosslinking type water absorptive polymer and water but not containing a flocculant aid, a flocculant, an agglomeration aid, a dry binder, a dry binding agent, a dry binding material, a sticky raw material, a thickener, an excipient, an alcohol, a crosslinking agent and a plasticizer and having a water content of from 1 to 20 % by weight and a water mobility value of less than 0.01 to a contact treatment with an oxidizing gas under circumstances at 0 °C or higher, regulating a temperature rise of the reaction mixture at 1 °C or higher within 10 minutes, and adjusting the water content so as to contain surplus water having a water mobility value of from 0.01 to 20 and has moldability due to the surplus water as a connecting substance, in which the water in the heat generating composition does not function as a barrier layer; that the moldable heat generating composition is molded by in-mold compression by filling in a mold cavity of a mold cavity-provided die to a depth substantially the same as the depth of the mold cavity and compressing to a thickness of from 50 to 99.5 % against the depth of the mold cavity; and that the compressed body is nonflexible and has shape holding properties.

EP 1 782 773 A1

**Description**

[Technical Field]

The present invention relates to a heat generating body having a wettable compressed body selected from granules, pallets, tablets, slugs, and mixtures thereof accommodated therein.

[Background Art]

In recent years, various thermal materials are widely used as a simple treatment tool for stiff shoulders, neuralgia, muscular pain, and the like. Usually, such a thermal material is constituted such that an exothermic agent capable of generating heat upon contact with air is covered by a film having a prescribed air permeability and that the heat generation is held for a prescribed time by gradually reacting the exothermic agent with air, and this is provided for use by sticking to the skin, a clothing, or the like by an adhesive. As this exothermic agent, a mixed powder made of a mixture of an iron powder, active carbon, NaCl and water is usually used.

On the other hand, Patent Document 1 discloses a heat generating body prepared by adding a binding agent to a powder and applying a pressure thereto, thereby molding the mixture in a block form.

Furthermore, Patent Document 2 discloses a heat generating body in which a heating element is accommodated in an accommodating section such as a pocket.

Furthermore, Patent Document 2 discloses a heat generating body in which a heat generating composition made of a dry compressed body heating element having a binding agent such as a flocculant contained therein is accommodated therein.

Furthermore, Patent Document 3 discloses a heat generating body made of a mixture of an exothermic agent capable of generating heat upon contact with air and a water absorptive polymer and/or a second polymer other than the water absorptive polymer, in which the mixture is pressure integrated under a pressure of from 100 to 800,000 kg/cm$^2$ together with any one of an alcohol, a crosslinking agent or a plasticizer, and a heat generating body as prepared by pressure integrating the mixture upon irradiation with light or heating.

However, when a powder is used, since the powder moves within a bag, it is difficult to form a thermal material having a uniform thickness. Thus, there was involved a problem that a uniform thermal effect is not obtained over the whole of the sticking surface. Furthermore, since the thermal material inevitably becomes thick as a whole, it was difficult to stick it on the entire surface in a portion with a large curvature. In particular, in the case where the thermal material is applied to a face or the like, there was involved a problem that the thermal material is so heavy that it deteriorates a feeling for use.

Furthermore, since the heat generating body in a block form as described in Patent Document 1 does not have flexibility at all, it is difficult to stick it to a curved part. Also, since this heat generating body is hard and brittle, when an impact is applied during the transportation or handling, there was involved a problem that an edge is cracked or broken.

Furthermore, in the heat generating body as described in Patent Document 2, a dry compressed body heating element is accommodated in an accommodating section such as a pocket, and a thermal cell is then exposed to oxygen, thereby causing heat generation. Thus, under the pretext of activation of the heat generating body, water or a salt solution must be injected into a hole of the central part or a water storage part of a dry compressed body heating element such as a tablet, or into a granulate composition through an oxygen-permeable heat generating body forming material sheet by an injection needle, resulting in a problem in productivity. Moreover, since a binding agent such as a flocculant is contained, there was involved a problem that an exothermic performance drops.

Furthermore, in the pressure integrated heat generating body as described in Patent Document 3, since the pressure integration is achieved under a pressure of from 100 to 800, 000 kg/cm$^2$ together with any one of an alcohol, a crosslinking agent or a plasticizer, the performance of the pressure integrated heat generating body varies depending upon the production conditions such as pressurization, heating and light irradiation conditions, resulting in a problem in stabilization of product quality. Thus, when this pressure integrated heat generating body is used as a general heat generating body, there was involved a problem in practical use in view of the exothermic duration, stability of product quality, and so on.

[Patent Document 1] JP-A-59-189183
[Patent Document 2] JP-T-11-508314
[Patent Document 3] WO 00/13626

[Disclosure of the Invention]

[Problems that the Invention is to Solve]

An object of the invention is to provide a wettable heat generating composition compressed body which has various shapes and various sizes, is wettable and is capable of generating heat upon contact with air. Another object of the invention is to provide a heat generating body having a structure in which the foregoing wettable heat generating composition compressed body is interposed between a substrate and a covering material and at least the periphery of the wettable heat generating composition compressed body is sealed and having an exothermic part having an air-permeable part. A further object of the invention is to provide a heat generating body which is flexible so that it is able to be fitted even to any curved portion of a human body, is free from an uncomfortable feeling at the time of fitting and is excellent in usefulness by constituting the foregoing exothermic part by an exothermic part composed of plural compartments. A still further object of the invention is to provide a lightweight and thin-layered heat generating body which is fitting to the whole of the face.

[Means for Solving the Problems]

In order to solve the foregoing problems, the present inventors made extensive and intensive investigations. As a result, they have developed a wettable heat generating composition compressed body having specific physical dimensions and filling characteristics and being able to form an exothermic part or including a specific iron oxidation chemical reaction to be put into an exothermic part, a heat generating body utilizing the same, and a process for producing for the foregoing wettable heat generating composition compressed body or heat generating body.

As set forth in claim 1, a wettable heat generating composition compressed body of the invention is a wettable heat generating composition compressed body resulting from compression of a moldable heat generating composition capable of causing an exothermic reaction upon contact with air, which is characterized in that:

1) the heat generating composition is one prepared by subjecting a reaction mixture containing, as essential components, an iron powder, a carbon component, a reaction accelerator, a crosslinking type water absorptive polymer and water but not containing a flocculant aid, a flocculant, an agglomeration aid, a dry binder, a dry binding agent, a dry binding material, a sticky raw material, a thickener, an excipient, an alcohol, a crosslinking agent and a plasticizer and having a water content of from 1 to 20 % by weight and a water mobility value of less than 0.01 to a contact treatment with an oxidizing gas under circumstances at 0 °C or higher, regulating a temperature rise of the reaction mixture at 1 °C or higher within 10 minutes, and adjusting the water content so as to contain surplus water having a water mobility value of from 0.01 to 20 and has moldability due to the surplus water as a connecting substance, in which the water in the heat generating composition does not function as a barrier layer; that
2) the moldable heat generating composition is molded by in-mold compression by filling in a mold cavity of a mold cavity-provided die to a depth substantially the same as the depth of the mold cavity and compressing to a thickness of from 50 to 99.5 % against the depth of the mold cavity; and that
3) the compressed body is non-flexible and has shape holding properties.

Also, a wettable heat generating composition compressed body as set forth in claim 2 is characterized in that in the wettable heat generating composition compressed body as set forth in claim 1, the heat generating composition contains at least one member selected from additional components consisting of a water retaining agent, a water absorptive polymer, a pH adjusting agent, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof.

Also, a wettable heat generating composition compressed body as set forth in claim 3 is characterized in that in wettable heat generating composition compressed body as according to claim 1, characterized in that the iron powder comprising particles, a surface of each of which is at least partially covered with an iron oxide film; that the iron oxide film has a thickness of 3 nm or more; and that the active iron powder particles having a region of an oxygen-free iron component in at least one region selected from a central region of the iron powder particles and a region beneath the iron oxide film is contained in an amount of from 20 to 100 % by weight.

As set forth in claim 4, a heat generating body of the invention is characterized by having an exothermic part having a structure in which the wettable heat generating composition compressed body as set forth in claim 1 is interposed between a substrate and a covering material and at least the periphery of the wettable heat generating composition compressed body is sealed.

Also, a heat generating body as set forth in claim 5 is

characterized in that in the heat generating body as set forth in claim 4, the exothermic part is an exothermic part in which a plural number of sectional exothermic parts are provided at intervals; that the sectional exothermic parts contain the wettable heat generating composition compressed body; that the wettable heat generating composition compressed body has a height of from 0.1 to 10 mm and a volume of from 0.01 to 30 $cm^3$; and that a ratio of the capacity of the sectional exothermic parts to the voleme of the wettable heat generating composition compressed body is from 0.6 to 1.0. Also, a heat generating body as set forth in claim 6 is characterized in that in the heat generating body as set forth in claim 4, in the heat generating body provided with two or more of the sectional exothermic parts, the air-permeable surface is covered by an air permeability adjusting material.

Also, a heat generating body as set forth in claim 7 is characterized in that in the heat generating body as set forth in claim 4, an outer periphery of the sealed wettable heat generating composition compressed body is collapsed by an outer pressure.

Also, a heat generating body as set forth in claim 8 is characterized in that in the heat generating body as set forth in claim 4, at least a part of one of the exposed surfaces of the heat generating body has a fixing measure.

As set forth in claim 9, a process for producing a wettable heat generating composition compressed body of the invention is a process for producing a wettable heat generating composition compressed body resulting from compression of a moldable heat generating composition capable of causing an exothermic reaction upon contact with air, which is characterized by:

1) using, as a heat generating composition, a moldable heat generating composition prepared by subjecting a reaction mixture containing, as essential components, an iron powder, a carbon component, a reaction accelerator, a crosslinking type water absorptive polymer and water but not containing a flocculant aid, a flocculant, an agglomeration aid, a dry binder, a dry binding agent, a dry binding material, a sticky raw material, a thickener, an excipient, an alcohol, a crosslinking agent and a plasticizer and having a water content of from 1 to 20 % by weight and a water mobility value of less than 0.01 to a contact treatment with an oxidizing gas under circumstances at 0 °C or higher, regulating a temperature rise of the reaction mixture at 1 °C or higher within 10 minutes, and adjusting the water content so as to contain surplus water having a water mobility value of from 0.01 to 20, and
2) filling the moldable heat generating composition in a mold cavity of a mold cavity-provided die to a depth substantially the same as the depth of the mold cavity and compressing to a thickness of from 50 to 99.5 % against the depth of the mold cavity.

Also, in the wettable heat generating composition compressed body, it is preferable that the iron powder contains from 20 to 100 % by weight of an active iron powder in which at least a part of the surface thereof is covered by a wustite film and an amount of the wustite is from 2 to 50 % by weight in terms of an X-ray peak intensity ratio to iron.

Also, it is preferable that the substrate and the covering material prior to sealing the periphery of the wettable heat generating composition compressed body are substantially flat and do not have a pocket, an accommodating section or an accommodating zone.

Also, it is preferable that in the heat generating body, at least one member of the substrate and the covering material prior to sealing the periphery of the wettable heat generating composition compressed body has a pocket and that the heat generating composition compressed body is accommodated in the pocket.

Also, it is preferable that in the heat generating body, the seal part is formed by heat seal after temporary adhering by a sticky layer and then forming a temporary seal and that an adhesive component which constitutes the sticky layer and a component of a heat seal material which constitutes a heat seal layer are co-present in the heat seal part.

Also, it is preferable that in the heat generating body, after the heat seal, at least a part of the accommodated wettable heat generating composition compressed body is transferred to the temporary adhering part which is not heat sealed, thereby deadhering the temporary adhering part which is not heat sealed.

Also, it is preferable that the heat generating body is provided with a perforation between the sectional exothermic parts.

Also, it is preferable that in the heat generating body, the fixing measure is an adhesive layer, and the adhesive layer contains at least one member selected from additional components consisting of a water retaining agent, a water absorptive polymer, a pH adjusting agent, a surfactant, an organosilicon compound, a hydrophobic polymer compound, a pyroelectric substance, an antioxidant, an aggregate, a fibrous material, a moisturizer, a functional substance, and a mixture thereof.

[Advantages of the Invention]

In the wettable heat generating composition compressed body of the invention, since a heat generating composition having a low water content is subjected to a contact treatment with an oxidizing gas, water in a necessary amount for obtaining practically useful exothermic characteristics is then added, and the mixture is compressed into a prescribed thickness, the wettable heat generating composition compressed body of the invention is able to take various shapes and various thicknesses, is of a thin type and is excellent in exothermic rising properties.

Furthermore, by providing plural compressed bodies of a small size made of a wettable heat generating composition compressed body at intervals, it is possible to provide a heat generating body which is able to be easily fitted to any curved part of a human body and can effectively impart a thermal effect to the whole of a sticking part.

Furthermore, exothermic characteristics including exothermic rising properties are extremely excellent.

Furthermore, not only the manufacturing costs of a heat generating body can be reduced, but also a lightweight and thin-layered heat generating body which is free from a stiff feeling, excellent in flexibility and extremely good in usefulness can be provided.

In the light of the above, in particular, the heat generating composition compressed body constituting a sectional exothermic part can be easily incorporated into a throwaway body wearing implement which can be adapted with various outer shapes of the body. Accordingly, it is possible to warm the body conveniently, comfortably and constantly.

[Best Modes for Carrying Out the Invention]

The wettable heat generating composition compressed body of the invention is a wettable heat generating composition compressed body resulting from compression of a moldable heat generating composition capable of causing an exothermic reaction upon contact with air, which is characterized in that:

1) the heat generating composition is one prepared by subjecting a reaction mixture containing, as essential components, an iron powder, a carbon component, a reaction accelerator, a crosslinking type water absorptive polymer and water but not containing a flocculant aid, a flocculant, an agglomeration aid, a dry binder, a dry binding agent, a dry binding material, a sticky binder, a thickener, an excipient, an alcohol, a crosslinking agent and a plasticizer and having a water content of from 1 to 20 % by weight and a water mobility value of less than 0.01 to a contact treatment with an oxidizing gas under circumstances at 0 °C or higher, regulating a temperature rise of the reaction mixture at 1 °C or higher within 10 minutes, and adjusting the water content so as to contain surplus water having a water mobility value of from 0.01 to 20 and has moldability due to the surplus water as a connecting substance, in which the water in the heat generating composition does not function as a barrier layer; that

2) the moldable heat generating composition is molded by in-mold compression by filling in a mold cavity of a mold cavity-provided die to a depth substantially the same as the depth of the mold cavity and compressing to a thickness of from 50 to 99.5 % against the depth of the mold cavity; and that

3) the compressed body is non-flexible and has shape holding properties.

By regulating the thickness of the heat generating composition compressed body at a depth of the mold cavity or making it on a basis of the depth, not only the degree of compression can be easily adjusted, but also the shape holding properties can be kept without scarifying exothermic characteristics such as exothermic rising properties, exothermic durability, and exothermic optimum temperature holding properties. The resistance to compression of the moldable heat generating composition having resistance to compression according to the invention is preferably 80 % or more, more preferably 85 % or more, and further preferably 90 % or more. The resistance to compression may be 100 % or more.

On the other hand, in the case of adding a flocculant aid, a flocculant, an agglomeration aid, a dry binder, a dry binding agent, a dry binding material, a sticky binder, a thickener, an excipient, an alcohol, a crosslinking agent or a plasticizer or determining the thickness of the compressed body by applying a pressure on a basis of a pressure, the exothermic characteristics, particularly exothermic rising properties are remarkably deteriorated, and it takes a long period of time for arrival at a desired temperature. Thus, it becomes difficult to produce a practically useful heat generating body. Furthermore, while it is possible to make a heat generating body of a short-time type, it is difficult to prepare a compressed body capable of continuing the heat generation of one hour or more at an optimum temperature. When the heat generating body is compressed, though the shape holding properties are improved, the exothermic rising properties are deteriorated, the exothermic maximum temperature drops, and the exothermic time becomes short. In particular, in a heat generating body whose flexibility is enhanced by adding a crosslinking agent, a plasticizer, etc., there was the case where for the purpose of increasing the exothermic characteristics, the exothermic body is inevitably used in an open state in which the air permeability is not adjusted. It was difficult to embody a heat generating body in which the air permeability is adjusted by using an air-permeable film, etc. and which is able to be mildly warmed over a long period of time.

Though the wettable heat generating composition compressed body of the invention is not flexible, after compression, it is a wetting compressed body capable of generating heat upon contact with air without adding water or a metal salt-containing aqueous solution to the compressed body, from which various heat generating bodies including heat generating bodies having one exothermic part and heat generating bodies having an exothermic part provided with plural compartments at intervals, all of which are free from pollution of the environment by a carbon component, etc. ,

have excellent exothermic characteristics and shape holding properties and are thoroughly durable against processing to a heat generating body. That is, it is possible to produce wettable heat generating composition compresses bodies of various shapes including from a curved shape to a linear shape and various sizes including from a small size to a large size, from an extra-thin size to a thick size, and from a narrow width to a wide width. Following this, it is possible to produce exothermic parts and heat generating bodies of various shapes and various sizes.

The sectional exothermic part or wettable heat generating composition compressed body of the invention usually has a maximum width of from 0.5 to 60 mm, preferably from 0.5 to 50 mm, more preferably from 1 to 50 mm, further preferably from 3 to 50 mm, still further preferably from 3 to 30 mm, even further preferably from 5 to 20 mm, even still further preferably from 5 to 15 mm, and even more still further preferably from 5 to 10 mm. Furthermore, its maximum height is usually from 0.1 to 30 mm, preferably from 0.1 to 10 mm, more preferably from 0.3 to 10 mm, further preferably from 1 to 10 mm, and still further preferably from 2 to 10 mm. Furthermore, its longest length is usually from 5 to 300 mm, preferably from 5 to 200 mm, more preferably from 5 to 100 mm, further preferably from 20 to 150 mm, and still further preferably from 30 to 100 mm.

Here, since the moldable heat generating composition having a water mobility value of from 0.01 to 20 according to the invention contains surplus water, when a pressure is applied, particles are readily brought into contact with each other, whereby the particles are fixed by means of a surface tension of water. Furthermore, since the wettable heat generating composition compressed body of the invention holds the thickness at the time of molding, namely the thickness of from 45 to 99. 5 % of the depth of the mold cavity, it is possible to sufficiently secure an exothermic duration without losing the water necessary for the heat generation at the time of compression and without need of activation by online or offline addition of water or a salt solution after the compression. Furthermore, although a rate of compression is from 50 to 99.5 % of the die thickness, it is preferably from 50 to 95 %, more preferably from 55 to 95 %, further preferably from 60 to 95 %, and still further preferably from 70 to 90 % of the die thickness.

Furthermore, the surplus water having a water mobility value of from 0.01 to 20 which is necessary in the invention was unpredictable. That is, there could be thought some possibility that surplus water covers the powder surface and functions as a barrier, whereby the exothermic reaction is remarkably deteriorated and that a prescribed amount of water must be removed from the heat generating composition as the case may be. However, since the surplus water having a water mobility value of from 0.01 to 20 is optimum in terms of surplus water, it imparted functions to the heat generating composition such that coupling of carbon and iron is promoted, a stiff compressed body is formed without excessively diluting the heat generating composition, and an exothermic reaction is caused immediately upon contact with air.

In the invention, by using surplus water having a water mobility value of from 0.01 to 20 in the compression operation, it is possible to reduce a carbon dust, to solve various problems in the manufacture, to increase the manufacture line speed and precision of charging weight, to improve fluidity of the heat generating composition, to eliminate non-uniformity of the heat generating composition within the completed exothermic part, to improve the performance of the completed exothermic part and to exclude necessity of special devices and circumstances, thereby remarkably lowering required labors, health, danger in safety and entire manufacture costs.

The exothermic part or heat generating body into which the heat generating composition compressed body capable of generating heat on a basis of a specific iron oxidation chemical reaction, which is produced by the production process of the invention, is incorporated has specific physical dimension and shape characteristics and gives long-term durable exothermic properties and improved temperature control properties. The sectional exothermic part and exothermic part contain a wet type compressed granulate exothermic substance or a wettable heat generating composition compressed body. This wet type compressed granulate exothermic substance or wettable heat generating composition compressed body substantially fills an effective exothermic part capacity in the sectional exothermic part and exothermic part and reduces an excessive blank capacity which is possibly present, thereby minimizing an ability of the exothermic substance to move within the exothermic part. This is achieved without necessity for applying a differential pressure to the exothermic part wall. Since these exothermic parts have a flexible physical dimension, when they are incorporated into a throwaway body wearing implement, etc., they can be made adaptive with various outer shapes of the body, whereby it becomes possible to warm the body conveniently, comfortably and constantly.

Accordingly, the invention is aimed to provide a wet compressed body and to provide a process for producing a wettable heat generating composition compressed body which directly uses compression of a powdered component including a carbonaceous material and iron, is able to rapidly reach a maximum temperature, continuously gives a controlled temperature, and is to be incorporated into a sectional exothermic part or an exothermic part. By incorporating such a wettable heat generating composition compressed body into a throwaway body wearing implement which can be adapted with various outer shapes, it becomes possible to warm the body conveniently, comfortably and constantly.

The wettable heat generating composition compressed body of the invention is one resulting from compression of a heat generating composition containing, as essential components, an iron powder, a carbon component, a reaction accelerator, a crosslinking type water absorptive polymer and water and containing surplus water having a water mobility value of from 0.01 to 20 to a thickness at the time of molding, namely a thickness of from 50 to 99.5 % of the depth of a mold cavity and is characterized by generating heat upon contact with air. That is, in the wettable heat generating composition compressed body of the invention, a wettable heat generating composition compressed body of a fixed shape or a sheet-like wettable heat generating composition compressed body can be obtained by filling a heat generating composition containing, as essential components, an iron powder, a carbon component, a reaction accelerator, a crosslinking type water absorptive polymer and water and containing surplus water having a water mobility value of from 0.01 to 20 in a mold cavity of a die and applying a prescribed pressure by using a compressing tool such as a compression plate.

Examples of a process for producing a heat generating body using the thus produced wettable heat generating composition compressed body include a production process in which the wettable heat generating composition compressed body is laminated on a substantially flat substrate, a covering material is covered thereon, and the periphery of the wettable heat generating composition compressed body is then heat sealed; and a production process in which the wettable heat generating composition compressed body is filled in a pocket of a pocket-provided packaging material, other packaging material is covered thereon, and the periphery of the pocket is then heat sealed. In the wettable heat generating composition compressed body, a shape necessary for filling it in a pocket can be sufficiently kept. Furthermore, in the heat generating body made of an exothermic part having plural sectional exothermic parts (the term "plural" means two or more) according to the invention, the space between the sectional exothermic parts becomes flexible, and the flexibility as a heat generating body is high. Thus, it is possible to make the heat generating body of the invention fit to any curved part of a human body. Furthermore, since exothermic characteristics per unit weight are excellent, only a small amount of the exothermic agent component is required for the purpose of realizing exothermic characteristics comparable to those of conventional heat generating bodies. Thus, it becomes possible to realize a lightweight and thin-layered heat generating body so that the feeling for use is remarkably improved.

As other production process of a heat generating body using the wettable heat generating composition compressed body of the invention, a heat generating body can also be produced by using a magnetic force outputted from a magnet in the bottom of a die singly or using a magnetic force jointly with reduced pressure, holding a magnetic iron-containing wettable heat generating composition compressed body in a prescribed flat place of a substrate, placing a covering material on the substrate such that the compressed body is placed between these two sheets, and subsequently sealing the compressed body between the substrate and the covering material. Furthermore, as another process, a heat generating body can also be produced by using a magnetic force outputted from a magnet in the bottom of a die singly or using a magnetic force jointly with reduced pressure, holding a magnetic iron-containing wettable heat generating composition compressed body in a pocket as formed in advance in a substrate or a pocket as formed by reduced pressure, placing a covering material on the substrate such that the compressed body is placed between these two sheets, and subsequently sealing the compressed body between the substrate and the covering material.

The individual constitutions of the heat generating body of the invention will be hereunder described in detail. The heat generating composition which is used in the invention is a heat generating composition containing, as essential components, an iron powder, a carbon component, a reaction accelerator, a crosslinking type water absorptive polymer and water and containing surplus water having a water mobility value of from 0.01 to 20.

The heat generating composition is not limited so far as it is a heat generating composition containing, as essential components, an iron powder, a carbon component, a reaction accelerator and water but not containing a flocculant aid, a flocculant, an agglomeration aid, a dry binding material, a dry binding agent, a dry binder, an adhesive binder, a thickener and an excipient, containing surplus water having a water mobility value of from 0.01 to 20 and having moldability due to the surplus water, with the water in the heat generating composition not functioning as a barrier layer, and capable of generating heat upon contact with air.

Incidentally, in the invention, what water does not function as a barrier layer and causes an exothermic reaction upon contact with air means that water in a heat generating composition does not function as a barrier layer which is an air intercepting layer and immediately after the production of a heat generating composition, comes into contact with air, thereby immediately causing an exothermic reaction.

A particle size of the water-insoluble solid component which constitutes the moldable heat generating composition of the invention is not limited so far as the heat generating composition has moldability. In the case where any one of the sizes (length, width and height) of the heat generating composition molded body resulting from molding of the heat generating composition is small, when the particle size is made small, the moldability is improved.

In addition, what the particle size of the solid component which constitutes the moldable heat generating composition is made small is preferable in view of molding. A maximum particle size of the water-insoluble solid component exclusive of the reaction accelerator and water in the components which constitutes the moldable heat generating composition is preferably not more than 2.5 mm, more preferably not more than 930 $\mu$m, further preferably not more than 500 $\mu$m, still further preferably not more than 300 $\mu$m, even further preferably not more than 250 $\mu$m, and even still further preferably not more than 200 $\mu$m. Moreover, 80 % or more of the particle size of the solid component is usually not more than 500 $\mu$m, preferably not more than 300 $\mu$m, more preferably not more than 250 $\mu$m, further preferably not more than 200 $\mu$m, still further preferably not more than 150 $\mu$m, and even further preferably not more than 100 $\mu$m.

In addition, if desired, at least one member selected from additional components selected from a water retaining agent, a pH adjusting agent, a hydrogen formation inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof may be added to the heat generating composition.

The components which constitute the heat generating composition and the kinds of the components can be used singly or in combination.

Furthermore, in the heat generating composition or the like according to the invention, there is no particular limitation with respect to the blending proportion thereof. However, it is preferred to select the blending proportion such that the heat generating composition contains from 1.0 to 50 parts by weight of a carbon component, from 0.01 to 10 parts by weight of a water retaining agent, from 0.01 to 20 parts by weight of a water absorptive polymer, from 0.01 to 5 parts by weight of a pH adjusting agent, from 0.01 to 12 parts by weight of a hydrogen formation inhibitor, and from 1.0 to 50 parts by weight of a reaction accelerator based on 100 parts by weight of an iron powder and contains from 1.0 to 60 parts by weight of water so as to have a water mobility value of from 0.01 to 20 as a heat generating composition. In addition, the following substances may be added in the following blending proportion based on the iron powder to the heat generating composition. That is, examples include a metal other than iron in an amount of from 1.0 to 50 parts by weight; a metal oxide other than iron oxide in an amount of from 1.0 to 50 parts by weight; a surfactant in an amount of from 0.01 to 5 parts by weight; an anti-foaming agent in an amount of from 0.01 to 5 parts by weight; a hydrophobic polymer compound, an aggregate, a fibrous material, a pyroelectric substance, a far infrared ray radiating substance, a minus ion emitting substance, and an organosilicon compound each in an amount of from 0.01 to 10 parts by weight; a moisturizer, an active substance, a fertilizer component, and a heat generating aid each in an amount of from 0.01 to 10 parts by weight; and an acidic substance in an amount of from 0.01 to 1 part by weight. In the case of blending a magnetic body, its blending proportion may be properly determined depending upon the desire.

The reaction mixture and the heat generating mixture are the same as in the moldable heat generating composition.

Furthermore, the following are preferable in view of improving the exothermic rising properties of the heat generating composition.

1) A heat generating composition is formed by subjecting a mixture of the essential components of the heat generating composition, or to which are further added an acidic substances and other necessary components, to a contact treatment (for example, self heat generation) with an oxidizing gas or further subjecting it to adjustment of water content or further adding and mixing other components therein.

2) An active iron powder having an oxygen-containing film such as oxides on at least a part of the surface of an iron powder is used as the iron powder.

   a) An iron powder having an oxygen-containing film of iron having a thickness, as determined by the Auger electron spectroscopy, of 3 nm or more with respect to the surface of the iron power is used.
   b) An iron powder having a wustite content of from 2 to 50 % by weight in terms of an X-ray peak intensity ratio to iron is used.

3) A mixture of an active iron powder having an oxygen-containing film such as oxides on at least a part of the surface of an iron powder and an iron powder not having an oxygen-containing film is used as the iron powder.

A usual iron powder, an iron alloy powder, an iron powder having an oxygen-containing film on at least a part of the surface of the iron powder, and an active iron powder made of an iron alloy powder are preferable as the iron powder.

As the usual iron powder, a cast iron powder, an atomized iron powder, an electrolyzed iron powder, a reduced iron powder, and the like can be used. Examples of the iron alloy powder include ones containing, as an alloy component, a metal including the foregoing semi-conductors.

The "iron alloy" as referred to herein is an alloy of iron containing 50 % or more of iron. The alloy component is not particularly limited so far as it is a metal component including semiconductors other than iron and the iron component functions as a component of the heat generating composition, and examples thereof include silicon, zinc, aluminum, magnesium, manganese, nickel, and copper.

As the metal oxide other than the iron oxide, any substance may be employed so far as it does not hinder the oxidation of iron by an oxidizing gas. Examples thereof include manganese dioxide and cupric oxide.

Examples of the iron powder having an oxygen-containing film on at least a part of the surface of iron include:

1) an active iron powder prepared by subjecting a mixture of the essential components of the heat generating composition, or to which are further added an acidic substances and other necessary components, to a contact treatment with an oxidizing gas, thereby partially oxidizing the iron component and partially oxidizing at least the surface of the iron component;

2) an active iron powder having a wustite content of from 2 to 50 % by weight; and

3) a mixture of an active iron powder and an iron powder other than the active iron powder.

Of these, a mixture containing 60 % or more of an active iron powder and less than 40 % of an iron powder other than the active iron powder is preferably enumerated.

In the case of storing the heat generating composition which is treated with an oxidizing gas or the heat generating composition containing an active iron powder, or a material utilizing the same over a long period of time, it is preferred to combine a hydrogen formation inhibitor therewith. This is because in this way, a heat generating body having excellent exothermic characteristics, which is inhibited in the formation of hydrogen, is free from swelling of the outer bag at the time of storage, etc. and has satisfactory exothermic rising properties, is obtained.

A thickness of the iron oxide film which covers the surface of the iron powder is usually from 3 nm or more, preferably from 3 nm to 100 $\mu$m, more preferably from 30 nm to 100 $\mu$m, further preferably from 30 nm to 50 $\mu$m, still further preferably from 30 nm to 1 $\mu$m, even further preferably from 30 nm to 500 nm, and even still further preferably from 50 nm to 300 nm by using the Auger electron spectroscopy. When the thickness of the oxygen-containing film of iron is 3 nm or more, the thickness of the oxygen-containing film of iron makes it possible to exhibit an effect for promoting an oxygen reaction, and upon contact with an oxidizing gas such as air, it is possible to immediately initiate the oxidation reaction. When the thickness of the oxygen-containing film of iron is 100 $\mu$m or more, though there is some possibility that the exothermic time becomes short, such can be employed depending upon the utility.

An amount of FeO (wustite) which is contained in the iron component containing the foregoing prescribed amount of wustite is usually from 2 to 50 % by weight, preferably from 2 to 40 % by weight, more preferably from 2 to 30 % by weight, and further preferably from 6 to 30 % by weight in terms of an X-ray peak intensity ratio to iron. Even when the amount of wustite exceeds 50 % by weight, though the exothermic rising properties are satisfactory, an exothermic duration becomes short. When the amount of wustite is less than 2 % by weight, the exothermic rising properties become dull.

The thickness of the foregoing prescribed amount of an oxygen-containing film or the oxygen-containing film of an iron powder containing wustite and the wustite content are to be applied to the heat generating composition or heat generating composition molded body at the time of lamination.

In the iron powder or active iron powder in the oxidizing gas-treated heat generating composition of the invention, at least a part of the surface thereof is covered by an oxygen-containing film of iron. The degree of covering on the surface of the oxygen-containing film of iron is not limited so far as at least a part of the surface thereof is covered, and the surface may be entirely covered. In the case of the heat generating composition of the invention, since an ion of the reaction accelerator such as a chlorine ion is contained in the heat generating composition, there is no corrosion effect of the oxide film due to a corrosion effect by the ion of the reaction accelerator such as a chlorine ion. Thus, the oxidation reaction which is a sort of corrosion is not hindered. In particular, in the case where an oxygen-containing film of iron is prepared while the ion of the reaction accelerator such as a chlorine ion exists together, the subject effect is large. In the case where a metal other than iron is present on the surface, it is only required that at least a part of the portion other than the metal other than iron is covered by the oxygen-containing film of iron.

In the iron powder of the invention, not only a region where (1) entire (uniform) corrosion, (2) pitting or crevice corrosion, (3) stress corrosion cracking, or the like is generated, but also irregularities or crevices are formed. For that reason, it is assumed that the iron powder of the invention has hydrophilicity and oxidation catalytic properties (for example, FeO) in its own portion. In producing the heat generating composition, it is important that the iron powder has an oxygen-containing film in its own portion without relying upon mixing. In particular, in the iron component as prepared by contact treating the iron component and the reaction accelerator and water as essential components with an oxidizing gas, it is thought that a reaction active part composed mainly of an oxide, a hydroxide, a chlorine ion, a hydrogen ion, etc. is formed, whereby exothermic reactivity and hydrophilicity are improved and exothermic rising properties and moldability are remarkably improved.

Furthermore, an iron powder which contains a carbon component and/or is covered by a carbon component is preferable, and an iron powder in which the surface of the iron powder is partially covered by from 0.3 to 3.0 % by weight of a conductive carbonaceous substance is useful. Examples of the conductive carbonaceous substance include carbon black and active carbon; and examples of the iron powder include reduced iron powders, atomized iron powders, and sponge iron powders. In particular, the case where the conductive carbonaceous substance is active carbon and the iron powder is a reduced iron powder is useful as a heat generating body.

In the case of measuring the water mobility value of the heat generating composition in the heat generating body and the thickness and amount of wustite of the iron oxide film of iron powder in the mixture or the heat generating composition in the heat generating body, the heat generating composition or mixture may be measured according to the following items.

1) Water mobility value:

The heat generating composition is taken out from the heat generating body and measured according to the foregoing method of measuring a water mobility value.

2) Thickness and amount of wustite of iron oxide film of iron powder:

A measuring sample as prepared by dispersing the heat generating composition, the heat generating composition molded body, the heat generating composition compression molded body or the mixture in nitrogen-purged ion-exchanged water in a nitrogen atmosphere, separating the iron powder using a magnet and drying the iron powder in a nitrogen atmosphere is used.

The carbon component is not particularly limited so far as it contains carbon as a component. Examples thereof include carbon black, graphite, active carbon, carbon nanotubes, carbon nanohorns, and flullerenes. Carbon which has become conductive by doping or the like is also employable. There are enumerated active carbons as prepared from coconut shell, wood, charcoal, coal, bone carbon, etc. and carbons as prepared from other raw materials such as animal products, natural gases, fats, oils, and resins. In particular, active carbons having an adsorption retaining ability are preferable.
Furthermore, it is not always required that the carbon component is present alone. In the case where an iron powder containing the carbon component and/or covered by the carbon component is used in the heat generating composition, it is to be noted that the heat generating composition contains the carbon component even though the carbon component is not present alone.
The reaction accelerator is not particularly limited so far as it is able to promote the reaction of the heat generating substance. Examples thereof include metal halides, nitrates, acetates, carbonates, and metal sulfates. Examples of metal halides include sodium chloride, potassium chloride, magnetic chloride, calcium chloride, ferrous chloride, ferric chloride, sodium bromide, potassium bromide, ferrous bromide, ferric bromide, sodium iodide, and potassium iodide. Examples of nitrates include sodium nitrate and potassium nitrate. Examples of acetates include sodium acetate. Examples of carbonates include ferrous carbonate. Examples of metal sulfates include potassium sulfate, sodium sulfate, and ferrous sulfate.
As the water, one from a proper source may be employed. Its purity and kind and the like are not particularly limited.
In the case of the heat generating composition, the content of water is preferably from 1 to 70 % by weight, more preferably from 1 to 60 % by weight, further preferably from 7 to 60 % by weight, still further preferably from 10 to 50 % by weight, and even further preferably from 20 to 50 % by weight of the heat generating composition.
Furthermore, in the case of the reaction mixture or heat generating mixture prior to the contact treatment with an oxidizing gas, the content of water is preferably from 0.5 to 20 % by weight, more preferably from 1 to 20 % by weight, further preferably from 3 to 20 % by weight, and still further preferably from 4 to 15 % by weight of the reaction mixture or heat generating mixture.
The water absorptive polymer is not particularly limited so far as it is a resin having a crosslinking structure and having a water absorption magnification of ion-exchanged water of 3 times or more of the dead weight. Furthermore, a water absorptive polymer the surface of which is crosslinked may be employed. Conventionally known water absorptive polymers and commercial products may also be employed.
The water absorptive polymer is not particularly limited so far as it is a resin having a crosslinking structure and having a water absorption magnification of ion-exchanged water of 3 times or more of the dead weight. Furthermore, a water absorptive polymer the surface of which is crosslinked may be employed. Conventionally known water absorptive polymers and commercial products may also be employed.
Examples of the water absorptive polymer include poly(meth)acrylic acid crosslinked materials, poly(meth)-acrylic acid salt crosslinked materials, sulfonic group-containing poly(meth)acrylic ester crosslinked materials, polyoxyalkylene group-containing poly(meth)acrylic ester crosslinked materials, poly(meth)acrylamide crosslinked materials, crosslinked materials of a copolymer of a (meth)acrylic acid salt and a (meth)acrylamide, crosslinked materials of a copolymer of a hydroxyalkyl (meth) acrylate and a (meth)acrylic acid salt, polydioxolane crosslinked materials, crosslinked polyethylene oxide, crosslinked polyvinylpyrrolidone, sulfonated polystyrene crosslinked materials, crosslinked polyvinylpyridine, saponification products of a starch-poly(meth)acrylonitrile graft copolymer, starch-poly(meth)acrylic acid (salt) graft crosslinked copolymers, reaction products of polyvinyl alcohol and maleic anhydride (salt), crosslinked polyvinyl alcohol sulfonic acid salts, polyvinyl alcohol-acrylic acid graft copolymers, and polyisobutylene maleic acid (salt) crosslinked polymers. These water absorptive polymers may be used alone or in combination with two or more kinds thereof.
Of these water absorptive polymers, water absorptive polymers having biodegradation properties are not limited so far as they are a biodegradable water absorptive polymer. Examples thereof include polyethylene oxide crosslinked materials, polyvinyl alcohol crosslinked materials, carboxymethyl cellulose crosslinked materials, alginic acid crosslinked materials, starch crosslinked materials, polyamino acid crosslinked materials, and polylactic acid crosslinked materials.

The water retaining agent is not limited so far as it is able to retain water. Examples thereof include porous materials derived from plants having high capillary function and hydrophilicity such as wood meal, pulp powder, active carbon, saw dust, cotton cloth having a number of cotton fluffs, short fiber of cotton, paper dust, and vegetable materials, water-containing magnesium silicate based clay minerals such as active clay and zeolite, pearlite, vermiculite, silica based porous substances, coralline stone, and volcanic ash based substances (for example, terraballoon, *shirasu* balloon, and *taisetsu* balloon). In order to increase a water retaining ability and enhance a shape holding ability of such a water retaining agent, the water retaining agent may be subjected to a processing treatment such as baking and/or pulverization.

The pH adjusting agent is not limited so far it is able to adjust the pH. Examples thereof include alkali metal weak acid salts and hydroxides and alkaline earth metal weak acid salts and hydroxides such as $Na_2CO_3$, $NaHCO_3$, $Na_3PO_4$, $Na_2HPO_4$, $Na_5P_3O_{10}$, NaOH, KOH, $Ca(OH)_2$, $Mg(OH)_2$, and $Ca_3(PO_4)_2$.
The hydrogen formation inhibitor is not limited so far as it is able to inhibit the formation of hydrogen. Examples thereof include one member or two or more members selected from the group consisting of sulfur compounds, oxidizing agents, alkaline substances, sulfur, antimony, selenium, phosphorus, and tellurium. Incidentally, examples of sulfur compounds include compounds with an alkali metal or an alkaline earth metal, metal sulfides such as calcium sulfide, metal sulfites such as sodium sulfite, and metal thiosulfates such as sodium thiosulfate.

The aggregate is not limited so far as it is useful as a filler and/or is useful for making the heat generating composition porous. Examples thereof include fossilized coral (for example, coral fossil and weathered coral fossil) , bamboo charcoal, bincho charcoal, silica-alumina powders, silica-magnesia powders, kaolin, colloidal silica, pumice, silica gel, silica powders, mica powders, clays, talc, synthetic resin powders or pellets, foamed synthetic resins such as foamed polyesters or polyurethanes, diatomaceous earth, alumina, and cellulose powder.

The fibrous material is an inorganic fibrous material and/or an organic fibrous material. Examples thereof include rock wool, glass fibers, carbon fibers, metal fibers, pulps, papers, non-woven fabrics, woven fabrics, natural fibers such as cotton and hemp, regenerated fibers such as rayon, semi-synthetic fibers such as acetates, synthetic fibers, and pulverized products thereof.

The functional substance is not limited so far as it is a substance having any function. Examples thereof include at least one member selected from minus ion emitting substances and far infrared ray radiating substances. The minus ion emitting substance is not limited so far as it emits a minus ion as a result either directly or indirectly, and examples thereof include ferroelectric substances such as tourmaline, fossilized coral, granite, and calcium strontium propionate, and ores containing a radioactive substance such as radium and radon. The far infrared ray radiating substance is not limited so far as it radiates far infrared rays. Examples thereof include ceramics, alumina, zeolite, zirconium, and silica.

The surfactant includes anionic surfactants, cationic surfactants, nonionic surfactants, and ampholytic surfactants. Especially, nonionic surfactants are preferable, and examples thereof include polyoxyethylene alkyl ethers, alkylphenol-ethylene oxide adducts, and higher alcohol phosphoric acid esters.

The organosilicon compound is not limited so far as it is a compound having at least an Si-O-R bond and/or an Si-N-R bond and/or an Si-R bond. The organosilicon compound is in the form of a monomer, a lowly condensed product, a polymer, etc. Examples thereof include organosilane compounds such as methyltriethoxysilane; and dimethylsilicone oil, polyorganosiloxane, or silicone resin compositions containing the same.

The pyroelectric substance is not limited so far as it has pyroelectricity. Examples thereof include tourmaline, hemimorphic ores, and pyroelectric ores. Tourmaline or achroite which is a kind of tourmaline is especially preferable. Examples of the tourmaline include dravite, schorl, and elbaite.

The moisturizer is not limited so far as it is able to hold moisture. Examples thereof include hyaluronic acid, collagen, glycerin, and urea.

The fertilizer component is not limited so far as it is a component containing at least one of three elements of nitrogen, phosphorus and potassium. Examples thereof include a bone powder, urea, ammonium sulfate, calcium perphosphate, potassium chloride, and calcium sulfate.

The hydrophobic polymer compound is not limited so far as it is a polymer compound having a contact angle with water of 40° or more, preferably 50° or more, and more preferably 60° or more in order to improve the draining in the composition. The shape of the hydrophobic polymer compound is not limited, and examples thereof include powdery, particulate, granular, and tablet shapes. Examples of the hydrophobic polymer compound include polyolefins such as polyethylene and polypropylene, polyesters, and polyamides.

Examples of the heat generating aid include metal powders, metal salts, and metal oxides such as Cu, Mn, $CuCl_2$, $FeCl_2$, manganese dioxide, cupric oxide, triiron tetroxide, and mixtures thereof.

As the metal oxide other than iron oxide, any material can be employed so far as it does not hinder the oxidation of iron by an oxidizing gas, and examples thereof include manganese dioxide and cupric oxide.

The acidic substance may be any of an inorganic acid, an organic acid, or an acidic salt. Examples thereof include hydrochloric acid, sulfuric acid, nitric acid, acetic acid, oxalic acid, citric acid, malic acid, maleic acid, chloroacetic acid, iron chloride, iron sulfate, iron oxalate, iron citrate, aluminum chloride, ammonium chloride, and hypochlorous acid.

The wettable heat generating composition molded body or the sectional exothermic part is not limited. The shape may be a planar shape, and examples thereof include a circular shape, an elliptical shape, a polygonal shape, a star shape, and a flower shape. Also, the shape may be a three-dimensional shape, and examples thereof include a polygonal pyramidal shape, a conical shape, a frustum shape, a spherical shape, a parallelepiped shape, a cylindrical shape, a semi-pillar shape, a semicylindroid shape, a semicylidrical shape, a pillar shape, and a cylindroid shape. Furthermore, in these shapes, the corner may be rounded, thereby processing the corner in a curvilinear or curved state, or the central part may be provided with a concave.

Furthermore, with respect to the shape, a maximum width is usually from 0.5 to 60 mm, preferably from 0.5 to 50 mm, more preferably from 1 to 50 mm, further preferably from 3 to 50 mm, still further preferably from 3 to 30 mm, even further preferably from 5 to 20 mm, even further preferably from 5 to 15 mm, and even still further preferably from 5 to 10 mm. Furthermore, a maximum height is usually from 0.1 to 30 mm, preferably from 0.1 to 10 mm, more preferably from 0.3 to 10 mm, further preferably from 1 to 10 mm, and still further preferably from 2 to 10 mm. Furthermore, a longest length is usually from 5 to 300 mm, preferably from 5 to 200 mm, more preferably from 5 to 100 mm, further preferably from 20 to 150 mm, and still further preferably from 30 to 100 mm.

Next, the heat generating body of the invention will be described. In the heat generating body using the wettable heat generating composition compressed body of the invention, the wettable heat generating composition compressed body is usually interposed between a substrate and a covering material, and the periphery of the wettable heat generating composition compressed body is subjecting to seal (for example, heat seal and compression seal), thereby obtaining a heat generating body. At this time, at least a part of the heat generating body has air permeability. Still further, a heat generating body capable of generating heat upon contact with air having a different shape may be formed by covering the heat generating body by a different packaging material. Furthermore, there is employed a constitution in which an adhesive is provided on a part of at least one exposed surface of the heat generating body and when used, the heat generating body is stuck to the skin, a cloth (for example, underwear), etc. Incidentally, for the purpose of reinforcing the strength of an air-permeable film or air-impermeable film which is used as a substrate or a covering material and achieving heat insulation, a non-woven fabric may be provided outside.

By making the thickness of the respective wettable heat generating composition compressed bodies different, it is possible to make temperature characteristics such as exothermic time, exothermic rising properties and exothermic peak temperature of the wettable heat generating composition compressed bodies different. Thus, in the heat generating body having a sectional exothermic part, by providing sectional exothermic parts in which these wettable heat generating composition compressed bodies having a different thickness are properly disposed, sectional exothermic parts having different temperature characteristics such as exothermic time, exothermic rising properties and exothermic peak temperature from each other are made present. Thus, the exothermic time, exothermic rising properties and exothermic peak temperature of the sectional exothermic parts are altered, thereby obtaining a heat generating body having desired temperature distribution, exothermic time, exothermic rising properties and exothermic peak temperature. A method for making the thickness of the respective wettable heat generating composition compressed bodies different is not limited. Examples thereof include a method for making the thickness of the respective wettable heat generating composition compressed bodies different by compressing the wettable heat generating composition compressed bodies by using a compression machine having a different height of a pushing part, thereby making a degree of compression different; and a method for making the thickness of the respective wettable heat generating composition compressed bodies different by compressing the wettable heat generating composition compressed bodies by using rubber rolls having different surface elasticity, thereby making a degree of compression different.

With respect to the shape of the heat generating body, any shape is employable. Examples thereof include a rectangular shape, a circular shape, an elliptical shape, a polygonal shape, a broad bean-like shape, an eye mask-like shape, a paper lantern-like shape, a cocoon-like shape, a gourd-like shape, a rectangular shape with rounded corners, a square shape with rounded corners, an egg-like shape, a boomerang-like shape, a comma-shaped bead-like shape, a wing-like shape, a nose-like shape, a star-like shape, and a foot-like shape.

A raw material of the substrate or covering material is not limited so far as it functions as an accommodating bag of the heat generating composition. Usually, raw materials which are used in chemical body warmers or heat generating bodies can be used. Examples of the raw material include air-impermeable raw materials, air-permeable raw materials, water absorptive raw materials, non-water absorptive raw materials, non-extensible raw materials, extensible raw materials, stretchable raw materials, non-stretchable raw materials, foamed raw materials, non-foamed raw materials, non-heat sealable raw materials, and heat sealable raw materials. The raw material can be properly used depending upon a desired utility in a desired form such as films, sheets, non-woven fabrics, woven fabrics, and composites thereof. In general, the substrate is made of an air-impermeable film or sheet, and the covering material is made of an air-permeable film or sheet or non-woven fabric, and vice versa. The both may be air-permeable. As the underlay material, an air-permeable underlay material and an air-impermeable underlay material may be used for different purposes.

The packaging material of the accommodating bag may be of a single-layered structure or multilayered structure, and its structure is not limited. Furthermore, though the packaging material is composed of at least a substrate and a covering material, a packaging material for laminating the heat generating composition molded body is the substrate, and a packaging material for covering on the heat generating composition molded body is the covering material regardless of whether the packaging material is air-permeable or air-impermeable. An embodiment of a multilayered structure in which an air-impermeable packaging material is the substrate and an air-permeable packaging material is the covering material will be hereunder described as one example. That is, in this embodiment, the substrate is made of layer A/layer B, layer A/layer B/layer C, or layer A/layer B/layer C/layer D; and the covering material is made of layer F/layer G, layer E/layer F/layer G, or layer F/layer H/layer G. Examples of the layer A include thermoplastic resin films (for example, polyethylene), heat seal layers (for example, polyethylene and EVA), and water absorptive papers; examples of the layer B include non-woven fabrics of a thermoplastic resin (for example, nylons), non-water absorptive papers, water absorptive papers, thermoplastic resin films (for example, polyethylene films, polypropylene films, polyester films, and polyamide (for example, nylons) films), wicks (for example, non-water absorptive papers and water absorptive papers); examples of the layer C include adhesive layers, non-water absorptive papers, water absorptive papers, thermoplastic resin films (for example, polyethylene), non-slip layers, and non-woven fabrics of a thermoplastic resin (for example, polyesters and nylons); examples of the layer D include separators, thermoplastic resin films (for example, polyethylene), and non-woven fabrics; examples of the layer E include heat seal layers; examples of the layer F include porous films or perforated films made of a thermoplastic resin (for example, polyethylene), films made of a thermoplastic resin (for example, polyethylene), non-water absorptive papers, and water absorptive papers; examples of the layer G include non-woven fabrics of a thermoplastic resin (for example, polyesters and nylons); and examples of the layer H include non-water absorptive papers and water absorptive papers. Examples of the substrate or covering material include heat seal layer made of polyethylene obtained by using a metallocene catalyst/polypropylene film, polyethylene-made heat seal layer/polypropylene film, EVA-made heat seal layer/polypropylene film, EVA-made heat seal layer/polypropylene film/adhesive layer/separator, EVA-made heat seal layer/polyethylene film/nylon non-woven fabric, non-woven fabric/porous film, heat seal layer made of polyethylene obtained by using a metallocene catalyst/polyethylene film/nylon non-woven fabric, heat seal layer made of polyethylene obtained by using a metallocene catalyst/polypropylene film/polypropylene non-woven fabric, non-woven fabric/(paper and/or perforated (provided by a needle or laser) film)/porous film, non-woven fabric/ (paper and/or porous film) /perforated (provided by a needle or laser) film, and non-woven fabric/(paper and/or porous film)/non-woven fabric. A method for laminating the respective layers is not limited. The respective layers may be directly laminated; the respective layers may be laminated via an air-permeable adhesive layer or a laminating agent layer; and the respective layers may be laminated by hot melt extrusion or the like. Furthermore, in the invention, it is to be noted that polyethylene produced by using a metallocene catalyst is also included in the polyethylene.

For example, in the case of laminating the foregoing raw material such as non-woven fabrics and porous films via an air-permeable sticky layer, examples of a method for forming the air-permeable sticky layer include a method in which a sticky substance is fibrillated by an appropriate system such as a curtain spray system, a melt blow system or a slot spray system for blowing and spreading a sticky substance via hot air under heat melting and spread and accumulated on an appropriate supporting substrate made of a porous film, an air-permeable substrate, a separator, etc., thereby forming a porous sticky layer.

A thickness of each of the substrate, the covering material, the underlay material, and the raw material constituting the same varies depending upon the utility and is not limited. The thickness is usually from 5 to 5,000 $\mu$m, preferably from 10 to 500 $\mu$m, and more preferably from 20 to 250 $\mu$m.

The air-impermeable raw material is not limited so far as it is air-impermeable. Examples thereof include films, sheets or coatings made of a polymer (for example, polyethylene, polypropylene, nylons, polyacrylates, polyesters, polyvinyl alcohols, and ethylene-vinyl acetate copolymers) and laminates thereof with a metal (including a semiconductor) compound (for example, silicon oxide) or composite raw materials using the same.

Of the foregoing air-impermeable raw materials, examples of a film having high air impermeability include films provided with a single layer or multiple layers of a thin film having a metal including a semiconductor or a compound thereof provided on an air-impermeable raw material film. Examples of the metal including a semiconductor include silicon, aluminum, and alloys or mixtures containing such a metal. Examples of the metal (including a semiconductor) compound include oxides, nitrides and oxynitrides of the foregoing metals or alloys or mixtures. Examples of the layer include silicon oxide layers, aluminum oxide layers, and silicon oxynitride layers; layers obtained by laminating an arbitrary layer of these layers on a polyester-made film; and layers obtained by further laminating a stretched polyolefin film (for example, a biaxially stretched polypropylene film) thereon.

The air-permeable raw material is not limited so far as it is air-permeable. Examples thereof include air-permeable films (for example, porous films and perforated films); materials having air permeability by themselves (for example, papers and non-woven fabrics); materials prepared by laminating at least one of papers and air-permeable films and non-woven fabrics so as to have air permeability; materials prepared by providing an air-impermeable packaging material comprising a non-woven fabric having a polyethylene film laminated thereon with fine pores by using a needle, etc. so as to have air permeability; non-woven fabric whose air permeability is controlled by laminating a fiber and heat bonding under pressure; porous films; and materials prepared by sticking a non-woven fabric onto a porous film. The "perforated film" as referred to herein is a film prepared by providing an air-impermeable film (for example, polyethylene films) with fine pores by using a needle so as to have air permeability.

The air permeability is not limited so far as the heat generation can be kept. In the case of use in usual heat generation, the air permeability is usually from 50 to 10,000 $g/m^2/24$ hr, preferably from 70 to 5,000 $g/m^2/24$ hr, more preferably from 100 to 2,000 $g/m^2/24$ hr, and further preferably from 100 to 700 $g/m^2/24$ hr in terms of moisture permeability by the Lyssy method.

When the moisture permeability is less 50 $g/m^2/24$ hr, the heat value is small and a sufficient thermal effect is not obtained, and therefore, such is not preferable. On the other hand, when it exceeds 10,000 $g/m^2/24$ hr, the exothermic temperature is high so that a problem in safety may possibly be generated, and therefore, such is not preferable. However, there is no limitation even when the moisture permeability exceeds 10,000 $g/m^2/24$ hr depending upon the utility, or even in the use at a moisture permeability closed to the open system, according to circumstances.

The stretchable packaging material is not particularly limited so far as it is stretchable. That is, it is only required that the stretchable packaging material is stretchable as a whole. The stretchable packaging material may be formed of a single material or a composite material of stretchable substrates or a combination of a stretchable substrate and a non-stretchable substrate.

Examples of the stretchable packaging material include single materials (for example, natural rubbers, regenerated rubbers, synthetic rubbers, elastomers, and stretchable shape memory polymers) and mixtures thereof, mixed materials or blended materials of such a stretchable raw material and a non-stretchable raw material or fabrics constituted of a combination of these materials, films, yarns, strands, ribbons, tapes, and stretchable films with a scrim structure.

The porous film is not limited and can be properly selected among porous films obtained by stretching a film made of a polyolefin based resin (for example, polyethylene, linear low density polyethylene, and polypropylene) or a fluorine based resin (for example, polytetrafluoroethylene) and a filler.

The non-woven fabric is not limited. Single non-woven fabrics of a single fiber or composite fiber made of a material such as rayon, nylons (polyamides), polyesters, polyacrylates, polypropylene, vinylon, polyethylene, polyurethane, cupra, cotton, cellulose, and pulp, or laminates of blended or accumulated fiber layers of such fibers are useful. Furthermore, from the standpoint of production process, dry non-woven fabrics, wet non-woven fabrics, spunbonds, spunlaces, and the like can be used. Non-woven fabrics made of a composite fiber having a core-sheath structure are also useful. A non-woven fabric in the side which is brought into contact with the skin is preferably a napping (fluffy) non-woven fabric. Also, stretchable non-woven fabrics and non-stretchable non-woven fabrics are useful.

The water absorptive raw material is not particularly limited so far as it is a water absorptive film or sheet.

The water absorptive raw material is not particularly limited so far as it has water absorption properties consequently regardless of whether or not the raw material has water absorption properties by itself.

Specific examples thereof include water absorptive foamed films or sheets having water absorption properties (for example, foamed bodies of water absorptive foamed polyurethane, etc.) or papers, non-woven fabrics or woven fabrics formed of a fiber having water absorption properties, non-woven fabrics or woven fabrics containing a fiber having water absorption properties, and water absorptive materials such as water absorptive porous films or sheets. Besides, there are enumerated materials in which regardless of the presence or absence of water absorption properties, a water absorbing agent is contained, impregnated, kneaded, transferred or carried on a foamed film or sheet, a non-woven fabric, a woven fabric or porous film or sheet, thereby imparting or increasing water absorption properties; and materials in which regardless of the presence or absence of water absorption properties, a water absorptive raw material such as water absorptive foamed films or sheets, papers, non-woven fabrics, woven fabrics, and porous films or sheets as cut in a planar shape according to the invention is attached to one side or both sides of the material according to the invention, thereby imparting water absorption properties.

In particular, in the heat generating body of the invention, for the purpose of forming the plane which is brought into contact with the skin into a comfortable plane by imparting water absorption properties against sweat, etc., in order that in the case of sweating, the sweat is absorbed, it is preferable that a packaging material in the plane which is brought into contact with the skin is constituted of a packaging material using a non-woven fabric or a woven fabric containing, as the major component, a water absorptive fiber having a water retention of 20 % or more. Examples of the water absorptive fiber having a water retention of 20 % or more include cottons, silks, hemps, wools, polyacrylonitrile based synthetic fibers, polyamide based synthetic fibers, polyvinyl alcohol based synthetic fibers, acetate fibers, triacetate fibers, and regenerated fibers. In addition, non-woven fabrics having a highly water absorptive polymer held in a non-woven fabric can be used as the non-woven fabric having excellent water absorption properties. Incidentally, non-woven fabrics or woven fabrics containing such a fiber as the major component are relatively good with respect to the feeling against the skin.

In addition, highly water absorptive packaging materials having high absorption properties of sweat can be used as the packaging material. Examples thereof include non-woven fabrics containing a fiber whose surface is coated with a highly water absorptive resin, non-woven fabrics containing a hollow fiber having a number of fine pores on the surface thereof, and non-woven fabrics containing a fiber having a capillary action by forming a number of pouches or plural layers in the cross-sectional shape.

Besides, non-woven fabrics or films having a water absorptive inorganic compound held on a non-sticky surface of a packaging material can be used. Examples thereof include non-woven fabrics resulting from holding a powder (for example, diatomaceous earth, zeolite, and silica gel) on a non-woven fabric and films resulting from holding a relatively large amount of a powder (for example, silica and alumina) on a synthetic resin (for example, polyethylene).

Furthermore, as an example of a different heat generating body of the invention, a pocket heat generating body having specific physical dimension and filling characteristics, capable of continuously generating heat over a long period of time and having improved temperature control properties by incorporating the wettable heat generating composition compressed body into a pocket of a pocket-provided substrate may be formed. This pocket heat generating body contains a wettable heat generating composition compressed body on a basis of a specific iron oxidation chemical reaction. This wettable heat generating composition compressed body fills in an effective pocket capacity within the pocket heat generating body and reduces an excessive blank capacity which is possibly present, thereby minimizing an ability of the wettable heat generating composition compressed body or exothermic substance to move. Since such a pocket heat generating body has a flexible physical dimension, it can be easily incorporated into a body wearing implement or the like which can be adapted with various outer shapes of the body. Accordingly, it is possible to warm the body conveniently, comfortably and constantly. Examples thereof include heat cells and all heat generating bodies using the same as described in JP-T-11-508786, JP-T-11-508314, JP-T-11-512954, JP-T-2002-514104, JP-T-2003-509120, and JP-T-2001-5075930. These are useful in the invention, and the disclosures of the patent documents can be totally incorporated in this description by reference.

In this case, in the case where two or more plural pockets are provided at intervals, a perforation may be provided in at least one pocket.

Furthermore, with respect to the wettable heat generating composition compressed bodies in the pocket, at least one thickness may be different.

By making the thickness of the respective wettable heat generating composition compressed bodies different, it is possible make the temperature characteristics of the wettable heat generating composition compressed body, such as exothermic time, exothermic rising properties and exothermic peak temperature different. Thus, in the heat generating body having a pocket, by providing a pocket in which these wettable heat generating composition compressed bodies having a different thickness are properly disposed, sectional exothermic parts having different temperature characteristics such as exothermic time, exothermic rising properties and exothermic peak temperature from each other are made present, thereby obtaining a heat generating body having different exothermic time, exothermic rising properties and exothermic peak temperature among the pockets. A method for making the thickness of the respective wettable heat generating composition compressed bodies different is not limited. Examples thereof include a method for making the thickness of the respective wettable heat generating composition compressed bodies different by compressing the wettable heat generating composition compressed bodies by using a compression machine having a different height of a pushing part, thereby making a degree of compression different; and a method for making the thickness of the respective wettable heat generating composition compressed bodies different by compressing the wettable heat generating composition compressed bodies by using rubber rolls having different surface elasticity, thereby making a degree of compression different.

Although the compressed body per se of the invention does not have flexibility, it has integral properties. Thus, in a heat generating body having an exothermic part in which compressed body-containing sectional exothermic parts are provided at prescribed intervals, it is possible to stick it to even a curved part with a large curvature. In addition, different from the case of a powder, the heat generating body can be produced in a uniform thickness and the subject state can be held. Thus, it is possible to impart a uniform thermal effect in the whole of the sticking portion. Furthermore, since the heat generating body is free from the movement of a powder in a bag as in a powder, it is free from an uncomfortable feeling and has an extremely excellent feeling for use. In particular, since the exothermic temperature and exothermic time per a unit weight of the metal powder are extremely excellent as compared with conventional powdery thermal materials, not only it is possible to realize a light weight and thin-layered heat generating body, but also it is possible to omit an air-permeable film, a non-woven fabric, and the like. Thus, it is possible to obtain a lightweight and thin-layered thermal sheet with an excellent feeling for use. Incidentally, with respect to the utility of the heat generating body of the invention, it is not limited to a throwaway body warmer, an exothermic sheet for thermal remedy, etc., but various utilities can be thought. Examples thereof include exothermic sheets or thermal wet packs in which various drugs are contained in an adhesive for the purpose of percutaneously absorbing a medicinal component and cosmetics using a cosmetic gelling agent as an adhesive. While the heat generating body of a structure having an adhesive layer constituted of an adhesive as a fixing measure has been described, the adhesive is not always required in the invention. In that case, the thermal material may be fixed by a stretchable bandage or a hook-and-loop fastener such as Velcro. Incidentally, the thermal material of the invention is sealed in an outer bag which is an air-impermeable accommodating bag prior to the use and transported or stored.

The production process of the invention is a process of filling a moldable heat generating composition in a mold cavity, applying a pressure to the moldable heat generating composition within the mold cavity and compressing it to a thickness of from 45 to 99.5 % of the depth of the mold cavity. The pressure at the time of pressurization is not particularly limited so far as the moldable heat generating composition can be compressed to a prescribed thickness. Furthermore, a compression jig which is used for compression molding is not limited. Examples thereof include elastic flat plates or rolls and flat plates or rolls having a pushing part capable of being inserted into the mold cavity.

In the heat generating composition compressed body which is produced by the in-mold compression, at least one of the packaging materials is interposed between oxygen-permeable packaging materials, and its periphery is heat sealed. For example, the heat generating composition compressed body is laminated on a substantially flat substrate, an air-permeable covering material is covered thereon, and the periphery of the heat generating composition compressed body is then heat sealed, thereby producing a heat generating body. Furthermore, in another example, a substrate having a pocket is used, the heat generating composition compressed body is charged in the pocket, and a covering material is then placed so as to cover the heat generating composition compressed body and the substrate. The substrate and the covering material are sealed in the periphery of the pocket having the heat generating composition compressed body charged therein, and the sealed pocket may be then formed as a completed heat generating body by cutting off the substrate and the covering material. Alternatively, for example, it can be incorporated into a body wearing implement for knee, neck, back, etc. Such a heat generating body or implement is sealed in an air-impermeable outer bag and then transported or stored.

The heat generating composition compressed body is of an arbitrary standard tablet shape such as a shallow convex surface shape, a concave surface standard shape, a deep concave surface shape, a flat surface shape, a flat edge part, a capsule shape with an obliquely cut edge part, an egg-like shape, and a modified spherical shape. The production process of the heat generating composition compressed body is not limited to the foregoing in-mold compression process, and any process can be employed so far as the heat generating composition compressed body can be produced.

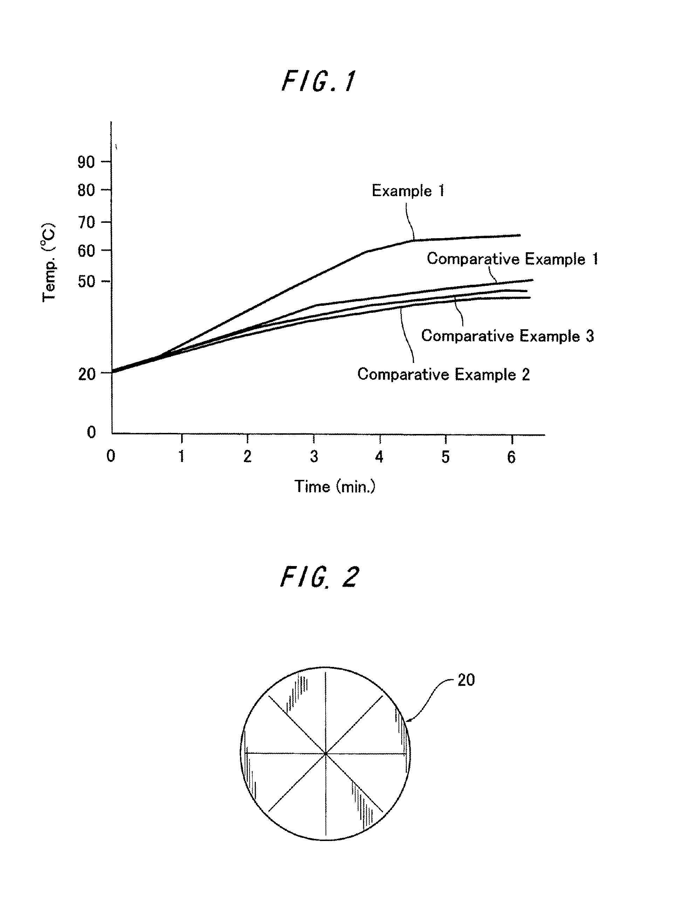

The "water mobility value" as referred to herein is a value showing an amount of surplus water which can transfer to the outside of the heat generating composition in water present in the heat generating composition. This water mobility value will be described below with reference to Figs. 2 to 6.

As shown in Fig. 2, a filter paper 20 of No. 2 (second class of JIS P3801) in which eight lines are drawn radiating from the central point with an interval of 45° is placed on a stainless steel plate 24 as shown in Figs. 3 and 4; a template 21 having a size of 150 mm in length $\times$ 100 mm in width and having a hollow cylindrical hole 22 having a size of 20 mm in inner diameter$\times$8 mm in height is placed in the center of the filter paper 20; a sample 23 is placed in the vicinity of the hollow cylindrical hole 22; and a stuffer plate 17 is moved on and along the template 21 and inserted into the hollow cylindrical hole 22 while stuffing the sample 23, thereby leveling the sample (force-in die molding).

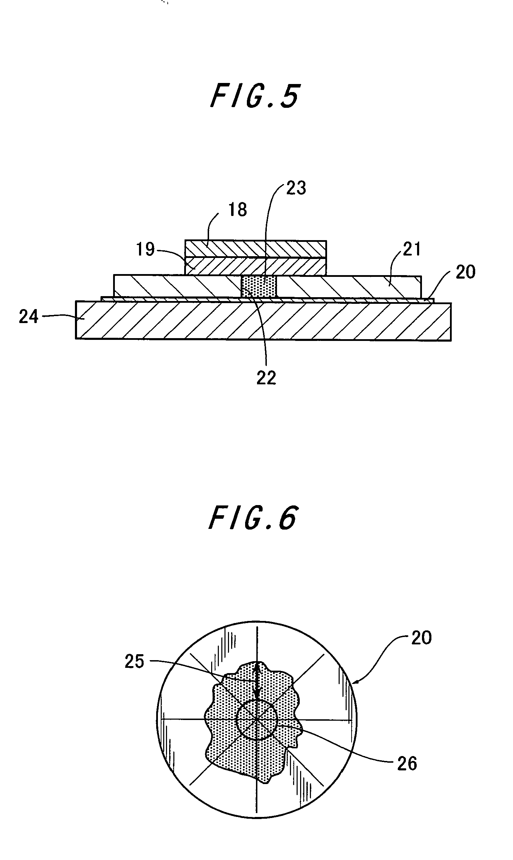

Next, as shown in Fig. 5, a non-water absorptive 70 $\mu$m-thick polyethylene film 19 is placed so as to cover the hole 22, and a flat plate 18 made of stainless steel having a size of 5 mm in thickness$\times$150 mm in length $\times$ 150 mm in width is further placed thereon and held for 5 minutes such that an exothermic reaction is not caused.

Thereafter, a shown in Fig. 6, the filter paper 20 is taken out, and an oozed-out locus of the water or aqueous solution is read as a distance 25 (unit: mm) from a periphery 26 as an edge of the hollow cylindrical hole to an oozed-out tip along the radiating lines. Similarly, a distance 25 from each of the lines is read, and eight values in total are obtained. Each of the eight values (a, b, c, d, e, f, g and h) which are read out is defined as a measured water content value. An arithmetic average value of the eight measured water content values is defined as a water content value (mm) of the sample.

Furthermore, the water content for the purpose of measuring a real water content value is defined as a compounded water content of the heat generating composition corresponding to the weight of the heat generating composition having a size of 20 mm in inner diameter $\times$ 8 mm in height or the like, similar measurement is conducted only with water corresponding to that water content, and a value as calculated in the same manner is defined as a real water content value (mm). A value obtained by dividing the water content value by the real water content value and then multiplying with 100 is a water mobility value.

That is, the water mobility value is represented by the following expression.

$$(\text{Water mobility value}) = \{[\text{Water content value (mm)}]/$$

$$[(\text{Real water content value (mm)})] \times 100$$

With respect to the same sample, five points are measured, and the five water mobility values are averaged, thereby defining an average value thereof as a water mobility value of the sample.

In the invention, a heat generating body can be formed only by laminating a heat generating composition molded body obtained by molding a heat generating composition having surplus water with a water mobility value of from 0.01 to 20 on a substrate, covering a covering material thereon, and sealing at least the periphery of the heat generating composition molded body. After accommodating it in a packaging material such as a substrate and a covering material, it is not necessary to add water. Accordingly, since the process is remarkably simplified, the invention is superior in view of the costs.

In the invention, the water mobility value (0 to 100) is preferably from 0.01 to 20, more preferably from 0.01 to 18, further preferably from 0.01 to 15, still further preferably from 0.01 to 13, even further preferably from 1 to 13, and even still further preferably from 3 to 13.

In a heat generating body using a heat generating composition molded body obtained by molding a moldable heat generating composition containing surplus water as a connecting substance according to the invention, the heat generating composition contains an appropriate amount of surplus water expressed by a water mobility value of from 0.01 to 20 as the connecting substance without using a flocculant aid, a dry binding agent, a flocculating agent, etc.

It is assumed that when the amount of surplus water in the heat generating composition is appropriate, the surplus water causes hydration against hydrophilic groups in the components of the composition due to a bipolar mutual action or hydrogen bond, etc. and that it is present even in the surroundings of hydrophobic groups while having high structural properties. Thus, it is assumed that the heat generating composition becomes in a state of a mud ball, thereby revealing moldability. This is connecting water as a connecting substance in some meaning. Besides, there is water in a state called as free water which can freely move, and it is thought that when the surplus water increases, the structure is softened, whereby the free water increases. Furthermore, controlling factors which an iron powder causes an oxidation reaction are an amount of existing water and a feed amount of oxygen to the surface of the iron powder. It is said that in a degree of water adsorbing film (less than 100 angstroms), the water is not sufficient and that the oxidation rate is small. When the adsorbing film becomes about 1 μm, the water content becomes sufficient. Furthermore, since the thickness of the water film is thin, feed of oxygen onto the surface of the iron powder becomes easy, whereby the oxidation rate becomes large. It is assumed that when the film becomes thicker to an extent that the adsorbing film exceeds 1 μm, the feed amount of oxygen is reduced. The present inventors have obtained knowledge that the water mobility value expressing the optimal water content at which moldability and oxidation rate in fixed levels or more are revealed is from 0.01 to 20, leading to accomplishment of the invention.

That is, by using an appropriate amount of surplus water, the respective component particles are coupled with each other by a surface tension of water, moldability is generated in the heat generating composition, and the water does not substantially function as a barrier layer. Thus, the heat generating composition comes into contact with air to generate heat. In addition, by using a heat generating composition using an active iron powder or an active heat generating composition using an active iron powder, the heat generating composition becomes a heat generating composition having remarkably excellent exothermic rising properties and high moldability. Furthermore, heat generation occurs without causing transfer of the water in the heat generating composition molded body as produced by a molding and laminating system into a packaging material or water absorptive sheet. In addition, by providing plural sectional exothermic parts of the heat generating composition molded body as sectioned by seal parts, it is possible to provide a heat generating body which has flexibility itself, is excellent in installation in places where flexibility is required, such as various places of a human body and curved bodies, and is excellent in feeling for use.

Furthermore, in the substrate, the covering material and the heat generating composition molded body, by temporarily adhering at least the covering material and the heat generating composition molded body to each other via a sticky layer and then heat sealing the periphery of the heat generating composition molded body and the surroundings of the heat generating body, certainty of heat seal is improved so that it becomes possible to design to make the production speed of a heat generating body high and make the heat seal width small.

The "moldability" as referred to in the invention exhibits that a molded body of the heat generating composition having a cavity or concave die shape is formed by force-through molding using a trimming die having a cavity or cast molding using a concave die, whereby after molding including mold release, the molding shape of the heat generating composition molded body is held.

When the moldability is revealed, since the shape is held until the heat generating composition molded article is at least covered by a covering material and a seal part is formed between the substrate and the covering material, sealing can be achieved in the periphery of the shape with a desired shape. Also, since so-called "spots" which are a collapsed piece of the heat generating composition are not scattered in the seal part, the sealing can be achieved without causing cutting in seal. The presence of the spots causes insufficient sealing.

1) Measurement device:

With respect to the measurement device, a stainless steel-made molding die (a plate having a size of 2 mm in thickness×200 mm in length×200 mm in width and having a cavity as treated by R5 in four corners of 60 mm in length× 40 mm in width in a central part thereof) and a fixable leveling plate are disposed above a travelable endless belt, and magnets (two magnets having a size of 12.5 mm in thickness× 24 mm in length×24 mm in width are disposed in parallel) are disposed under the endless belt.

The magnets should cover a region of the leveling plate and the vicinity thereof and a region larger than a region covered by a cut side (40 mm) vertical to the advancing direction of the cavity of the molding die.

2) Measurement method:

With respect to the measurement method, a stainless steel plate having a size of 1 mm in thickness×200 mm in length× 200 mm in width is placed on the endless belt of the measurement device, a polyethylene film having a size of 70 μm in thickness×200 mm in length×200 mm in width is placed thereon, and a stainless steel-made molding die is further placed thereon.

Thereafter, a leveling plate is fixed in a position of the cavity of the molding die of 50 mm far from the end portion in the advancing direction of the endless belt, 50 g of a heat generating composition is then placed in the vicinity of the leveling plate between the leveling plate and the cavity, and the heat generating composition is filled in the cavity of the molding die while leveling it by moving the endless belt at 1. 8 m/min. After the molding die has completely passed through the leveling plate, the traveling of the endless belt is stopped. Next, the molding die is removed, and a heat generating composition molded body as laminated on the polyethylene film is observed.

3) Judgment method:

With respect to the judgment method, in the surroundings of the heat generating composition molded body, in the case where any collapsed piece of the heat generating composition molded body exceeding a maximum length of 800 μm is not present and the number of collapsed pieces of the heat generating composition molded body having a maximum length of from 300 to 800 μm is not more than 5, it is to be noted that the heat generating composition has moldability.

The moldability is an essential property for a heat generating composition to be used in the molding system. If the heat generating composition does not have moldability, it is impossible to produce a heat generating body by the molding system.

The "adjustment of the water content" as referred to herein means that after contact treating the heat generating mixture with an oxidizing gas, water or an aqueous solution of a reaction accelerator is added. Although the amount of addition of water or an aqueous solution of a reaction accelerator is not limited, examples thereof include the addition of a weight corresponding to a reduced weight by the contact treatment and the addition of a weight such that a desired water mobility value is obtained.

Whether or nor the adjustment of the water content is introduced may be properly determined depending upon the utility.

The "moldability" as referred to in the invention exhibits that a laminate of the heat generating composition having a cavity or concave die shape can be formed by force-through molding using a trimming die having a cavity or cast molding using a concave die and after molding including mold release, the molding shape of the heat generating composition molded body is held. When the moldability is revealed, since the shape is held until the heat generating composition molded article is at least covered by a covering material and a seal part is formed between the substrate and the covering material, sealing can be achieved in the periphery of the shape with a desired shape. Also, since so-called "spots" which are a collapsed piece of the heat generating composition are not scattered in the seal part, sealing can be achieved without causing cutting in seal. The presence of the spots causes insufficient sealing.

Next, with respect to the moldability, a measurement device, a measurement method and a judgment method will be described below.

1) Measurement device:

With respect to the measurement device, a stainless steel-made molding die (a plate having a size of 2 mm in thickness×200 mm in length×200 mm in width and having a cavity as treated by R5 in four corners of 60 mm in length× 40 mm in width in a central part thereof) and a fixable leveling plate are disposed above a travelable endless belt, and magnets (two magnets having a size of 12.5 mm in thickness × 24 mm in length × 24 mm in width are disposed in parallel) are disposed under the endless belt. The magnets should cover a region of the leveling plate and the vicinity thereof and a region larger than a region covered by a cut side (40 mm) vertical to the advancing direction of the cavity of the molding die.

2) Measurement method:

With respect to the measurement method, a stainless steel plate having a size of 1 mm in thickness $\times$ 200 mm in length $\times$ 200 mm in width is placed on the endless belt of the measurement device, a polyethylene film having a size of 70 $\mu$m in thickness $\times$ 200 mm in length $\times$ 200 mm in width is placed thereon, and a stainless steel-made molding die is further placed thereon. Thereafter, a leveling plate is fixed in a position of the cavity of the molding die of 50 mm far from the end portion in the advancing direction of the endless belt, 50 g of a heat generating composition is then placed in the vicinity of the leveling plate between the leveling plate and the cavity, and the heat generating composition is filled in the cavity of the molding die while leveling it by moving the endless belt at 1.8 m/min.

After the molding die has completely passed through the leveling plate, the traveling of the endless belt is stopped. Next, the molding die is removed, and a heat generating composition molded body as laminated on the polyethylene film is observed.

3) Judgment method:

With respect to the judgment method, in the surroundings of the heat generating composition molded body, in the case where any collapsed piece of the heat generating composition molded body exceeding a maximum length of 800 $\mu$m is not present and the number of collapsed pieces of the heat generating composition molded body having a maximum length of from 300 to 800 $\mu$m is not more than 5, it is to be noted that the heat generating composition has moldability. The moldability is an essential property for a heat generating composition to be used in the molding system. If the heat generating composition does not have moldability, it is impossible to produce a heat generating body by the molding system.

The heat generating composition of the invention has resistance to compression. The "resistance to compression" as referred to herein means that a heat generating composition compressed body obtained by compressing a heat generating composition molded body as accommodated in a molding die within the die to such an extent that the thickness is 70 % of the die thickness holds 80 % or more of exothermic rising properties of the exothermic rising properties of the heat generating composition molded body before compression (a difference in temperature between one minute and 3 minutes after starting a heat generation test of the heat generating composition).

Here, the measurement method of exothermic rinsing properties for the resistance to compression will be described below.

1. Heat generating composition molded body:

1) A magnet is provided in the vicinity of a central part of the back side of a polyvinyl chloride-made supporting plate (3 mm in thickness$\times$600 mm in length$\times$600 mm in width) of a footed supporting table so as to cover a cavity shape of a molding die.

2) A temperature sensor is placed on the central part the surface of the supporting plate.

3) A polyethylene film (25 $\mu$m in thickness$\times$250 mm in length$\times$200 mm in width) as provided with an adhesive layer having a thickness of about 80 $\mu$m is stuck onto the supporting plate via a sticky layer such that the center of the polyethylene film is positioned at the sensor.

4) On an underlay plate (280 mm in length$\times$150 mm in width$\times$50 $\mu$m to 2 mm in thickness), a polyethylene film (230 mm in length$\times$155 mm in width$\times$25 $\mu$m to 100 $\mu$m in thickness) is placed such that one end of the polyethylene film is projected by about 20 mm outside the underlay plate and that one end thereof in the length direction is substantially coincident with one end of the underlay plate.

5) A template (230 mm in length$\times$120 mm in width$\times$3 mm in thickness) having a cavity (80 mm in length$\times$50 mm in width$\times$3 mm in height) is placed on the polyethylen film placed on the underlay plate; a template is placed on the polyethylene film such that one end thereof in the length direction is fitted to one end where the underlay plate and the polyethylene film are coincident with each other and that in the width direction, one end part of the width of the template is placed at a position of the central part by about 20 mm far from an opposing end to the side where the polyethylene film is projected outward from the underlay plate. Next, the resulting assembly is placed on the supporting plate together with the underlay plate.

6) A sample is placed in the vicinity of the cavity; a force-in die plate is moved along the molding die; the sample is charged into the cavity while stuffing; and the sample is leveled while stuffing along the template plane (force-in die molding), thereby filling the sample in the die.

7) Next, the magnet beneath the supporting plate is removed; the end portion of the projected polyethylene film is pressed; the underlay plate is removed; and the temperature measurement is started.

2. Heat generating composition compressed body:

1) to 6) are the same as in the case of the heat generating composition molded body.
8) A die having a convex having a thickness of 0.9 mm which can substantially tightly come into the cavity in relation of the cavity with an unevenness is fitted to the cavity and compressed by a roll press or plate press to prepare a heat generating composition compressed body having a thickness of 2.1 mm (compressed to 70 % of the die thickness) within the die.
9) The resulting assembly is placed on the supporting plate together with the underlay plate; the magnet beneath the supporting plate is removed; the end portion of the projected polyethylene film is pressed; the underlay plate is removed; and the temperature measurement is started.
With respect to the measurement of the exothermic temperature, the temperature is measured for 5 minutes at a measurement timing of 2 seconds using a data collector, and resistance to compression is judged in terms of a difference in temperature between after elapsing one minute and after elapsing 3 minutes.
The thickness after compression is preferably from 50 to 99.5 %, more preferably from 60 to 99.5 %, and further preferably from 60 to 95 % of the die thickness.
Incidentally, in the invention, it is to be noted that the heat generating composition molded body includes a heat generating composition compressed body.

The particle size of the water-insoluble solid component constituting the moldable heat generating composition of the invention is not limited so far as the heat generating composition has moldability. In the case where any one of length, width and height as the size of the heat generating composition molded body as molded from the heat generating composition is small, the moldability is improved by making the particle size small.
In addition, it is preferable in view of molding that the particle size of the solid component constituting the moldable heat generating composition is small. A maximum particle size of the water-insoluble solid component exclusive of the reaction accelerator and water in the components constituting the moldable heat generating composition is preferably not more than 2.5 mm, more preferably not more than 930 $\mu$m, further preferably not more than 500 $\mu$m, still further preferably not more than 300 $\mu$m, even further preferably not more than 250 $\mu$m, and even still further preferably not more than 200 $\mu$m. Moreover, 80 % or more of the particle size of the solid component is usually not more than 500 $\mu$m, preferably not more than 300 $\mu$m, more preferably not more than 250 $\mu$m, further preferably not more than 200 $\mu$m, still further preferably not more than 150 $\mu$m, and even further preferably not more than 100 $\mu$m.
Incidentally, with respect to the particle size of the water-insoluble solid component, separation is conducted using a sieve, and the particle size of the component which has passed through the sieve is calculated from an opening of the sieve. That is, sieves of 8, 12, 20, 32, 42, 60, 80, 100, 115, 150, 200, 250 and 280 meshes and a receiving dish are combined in this order from up to down. About 50 g of water-insoluble solid component particles are placed on the uppermost 8-mesh sieve and shaken for one minute using an automatic shaker. Weights of the water-insoluble solid component particles on each of the sieves and the receiving dish are weighed. The total amount thereof is defined as 100 %, and the particle size distribution is determined from weight fractions. When the sum of all receiving dishes under the sieve of a specific mesh size becomes 100 % which is the total sum of the particle size distribution, the size ($\mu$m) calculated from the opening of the specific mesh is defined as the particle size of the water-insoluble solid component.
Incidentally, each of the mesh sieves may be combined with other mesh sieves. Here, the particles which have passed through a 16-mesh sieve are defined to have a particle size of not more than 1 mm; the particles which have passed through a 20-mesh sieve are defined to have a particle size of not more than 850 $\mu$m; the particles which have passed through a 48-mesh sieve are defined to have a particle size of not more than 300$\mu$ m; the particles which have passed through a 60-mesh sieve are defined to have a particle size of not more than 250 $\mu$m; the particles which have passed through a 65-mesh sieve are defined to have a particle size of not more than 200 $\mu$m; the particles which have passed through an 80-mesh sieve are defined to have a particle size of not more than 180 $\mu$m; the particles which have passed through a 100-mesh sieve are defined to have a particle size of not more than 150 $\mu$m; the particles which have passed through a 115-mesh sieve are defined to have a particle size of not more than 120 $\mu$m; the particles which have passed through a 150-mesh sieve are defined to have a particle size of not more than 100 $\mu$m; and the particles which have passed through a 250-mesh sieve are defined to have a particle size of not more 63 $\mu$m, respectively. The same is applicable to mesh sizes of less than these mesh sizes.
Furthermore, the heat generating composition can be classified into a powder, a granulate heat generating composition (having a water mobility value of less than 0.01), a moldable heat generating composition (having a water mobility value of from 0.01 to 20), and a sherbet-like heat generating composition (having a water mobility value exceeding 20 but not more than 50) depending upon the state of adjustment of the water content or surplus water. The heat generating composition as classified depending upon the water mobility value is as described previously.
The "contact treatment with an oxidizing gas" as referred to herein is a method in which a mixture or heat generating composition having components of the heat generating composition mixed therein is brought into continuous or intermittent contact with an oxidizing gas (for example, oxygen and air) in an oxidizing gas atmosphere or by blowing an oxidizing gas or other means, thereby partially oxidizing the iron component. A method for determining a degree of oxidation is not limited. Examples thereof include a method in which a degree of contact of the mixture or heat generating composition with an oxidizing gas is determined by the water mobility value of the mixture or heat generating composition, the contact time with the oxidizing gas, the exothermic temperature rise rate at the time of contact, the exothermic temperature at the time of contact, the maximum exothermic temperature at the time of contact, a prescribed temperature as dropped after reaching the maximum exothermic temperature at the time of contact, or a combination thereof, thereby determining a degree of oxidation.

For examples, the following methods are preferable.

(1) A heat generating composition having a water mobility value of not more than 20 (for example, less than 0.01 or from 0.01 to 20) is exposed to air while fluidizing by stirring or the like to cause self heat generation, intercepted from air for a desired period of time until the temperature exceeds a maximum exothermic temperature and then returned to room temperature, thereby forming a heat generating composition. In particular, a contact treatment with an oxidizing gas by exposing a heat generating mixture or heat generating composition having a water mobility value of less than 0.01 to air while stirring, thereby causing self heat generation is preferable.
(2) A heat generating composition having a water mobility value exceeding 20 is brought into contact with air and intercepted from air for a desired period of time, thereby forming a heat generating composition.
(3) Water or a reaction accelerator aqueous solution is added to the heat generating composition as obtained in either one of (1) or (2), and the water content of the mixture is adjusted, followed by mixing to form a heat generating composition having a desired water mobility value. The weight of the water or reaction accelerator aqueous solution to be added for the purpose of adjusting the water content is not limited. Examples thereof include a weight as reduced against the weight of the mixture or heat generating composition prior to exposing to air, namely prior to causing self heat generation, or a weight corresponding to the weight exceeding it. If desired, the temperature state of the mixture and the heat generating composition may be controlled prior to the contact treatment and/or at the time of contact treatment by warming the mixture, warming the heat generating composition and warming a reaction vessel, heat insulation, cooling, or a combination thereof. In this way, a heat generating composition having remarkably excellent exothermic rising properties can be obtained.

The following methods are enumerated.

(1) A heat generating composition having a water mobility value of not more than 20 (for example, less than 0.01 or from 0.01 to 30) is exposed to air while fluidizing by stirring or the like to cause self heat generation, intercepted from air for a desired period of time until the temperature exceeds a maximum exothermic temperature and then returned to room temperature, thereby forming a heat generating composition. In particular, a contact treatment with an oxidizing gas by exposing a heat generating mixture or heat generating composition having a water mobility value of less than 0.01 to air while stirring, thereby causing self heat generation is preferable.
(2) A heat generating composition having a water mobility value exceeding 20 is brought into contact with air and intercepted from air for a desired period of time, thereby forming a heat generating composition.
(3) Water or a reaction accelerator aqueous solution is added to the heat generating composition as obtained in either one of (1) or (2), and the water content of the mixture is adjusted, followed by mixing to form a heat generating composition having a desired water mobility value. The weight of the water or reaction accelerator aqueous solution to be added for the purpose of adjusting the water content is not limited. Examples thereof include a weight as reduced against the weight of the mixture or heat generating composition prior to exposing to air, namely prior to causing self heat generation, or a weight corresponding to the weight exceeding it. If desired, the temperature state of the mixture and the heat generating composition may be controlled prior to the contact treatment and/or at the time of contact treatment by warming the mixture, warming the heat generating composition and warming a reaction vessel, heat insulation, cooling, or a combination thereof. In this way, a heat generating composition having remarkably excellent exothermic rising properties can be obtained.

As the "oxidizing gas" as referred to herein, any substance may be employed so far as it is gaseous and oxidizing. Examples thereof include an oxygen gas, air, and a mixed gas of an inert gas (for example, a nitrogen gas, an argon gas, and a helium gas) and an oxygen gas. Of these, air is especially preferable.

So far as the atmosphere of the contact treatment region does not become deficient in oxygen and an oxidation reaction of the iron component is caused, a temperature of the oxidizing gas, a temperature of the contact treatment and a time of the contact treatment are not limited and may be properly determined depending upon the desire. The temperature of the oxidizing gas is preferably from 0 to 200 °C, more preferably from 10 to 150 °C, and further preferably from 20 to 100 °C. Furthermore, the treatment time is preferably from one second to 10 minutes, more preferably from 5 seconds to 7 minutes, and further preferably from 15 seconds to 5 minutes. In the step, it is preferable that the reaction time is short.

The amount of the oxidizing gas to be used may be adjusted depending upon the kind of the oxidizing gas, the kind and particle size of the iron powder, the water content, the treatment temperature, the treatment method, and the like. In the case of using air, the amount of air is preferably from 1 to 1,000 liters/min per 200 g of the iron powder under one atmosphere at 100 °C. In the case of other oxidizing gas, the amount of the oxidizing gas may be reduced into the concentration of oxygen on the basis of the case of air.

If desired, an acidic substance or a peroxide may be added at the time of the contact treatment with an oxidizing gas. Examples of the peroxide include hydrogen peroxide and ozone.

The "iron oxide film" as referred to herein is a film made of oxygen-containing iron such as iron oxide, hydroxide or oxyhydroxide.

The "thickness of the iron oxide film" as referred to herein means a portion in which in the case of sputtering the surface of the iron powder with Ar at a sputtering rate of 11 nm/min as reduced into Fe in the depth direction by using the Auger electron spectroscopy, a ratio (Io/Ii) of a peak intensity of O (Io) to a peak intensity of Fe (Ii) is 0.05 or more. Accordingly, the thickness of the oxygen-containing film of iron of the invention is a distance, as reduced into Fe, from the surface of the iron powder to a depth at which (Io/Ii) is 0.05. With respect to the measurement condition of the Auger electron spectroscopy, the sputtering time is 15 minutes, and the sputtering rate is 11 nm/min (as reduced into Fe). With a lapse of the sputtering time in the Auger electron spectroscopy, Io decreases, whereas Ii increases. By reducing the sputtering time from the surface of the iron powder to a depth at which (Io/Ii) is 0.05 into a thickness, the thickness of the iron oxide film can be calculated.

The "amount of wustite" as referred to herein is an amount expressed by % according to the following expression from an integrated intensity of peaks of a (110) plane of iron (αFe) and an integrated intensity of peaks of a (220) plane of FeO (wustite) by using an X-ray diffraction device.

$$[Amount\ of\ wustite\ (\%)] = 100 \times KFeO/(K\alpha Fe)$$

KFeO: Integrated intensity of peaks of a (220) plane of FeO (wustite)
KαFe: Integrated intensity of peaks of a (110) plane of iron (αFe)

An amount of wustite is usually from 2 to 50 % by weight, preferably from 5.01 to 50 % by weight, more preferably from 5.01 to 40 % by weight, further preferably from 6 to 40 % by weight, still further preferably from 7 to 30 % by weight, and even further preferably from 7 to 25 % by weight. Even when the amount of wustite exceeds 50 % by weight, though the exothermic rising properties are satisfactory, an exothermic duration becomes short. When the amount of wustite is less than 2 % by weight, the exothermic rising properties become dull.

The "active iron powder" as referred to herein is an iron powder having a region where oxygen and iron are present and a region whose thickness is 3 nm or more by the Auger electron spectroscopy and where oxygen is not present or iron is present. Alternatively, the active iron powder is an iron powder having a content of wustite of from 2 to 50 % by weight in terms of an X-ray peak intensity ratio to iron. Furthermore, when the active iron powder is prepared by using a mixture containing an iron powder and at least any one of other essential components (for example, a carbon component, a reaction accelerator, and water), in the case where as a result of separating the iron powder from the mixture after the preparation by using a magnet, etc. and providing as a sample for measurement by the Augur electron spectroscopy, the iron powder has a region where oxygen and iron are present, a thickness of that region is 3 nm or more, and the iron powder has a region of an oxygen-free iron component in at least one region selected from a central part region of the iron powder where at least oxygen and iron are present and a region beneath the iron oxide film, the subject iron powder is defined to be an active iron powder. Alternatively, when as a result of determining the amount of wustite by using an X-ray diffraction device, it falls within the range of from 2 to 50 % by weight or more in terms of an X-ray peak intensity ratio to iron, the subject iron powder is defined to be an active iron powder.

The "heat generating mixture" as referred to herein is a material obtained by subjecting a reaction mixture containing, as essential components, an iron powder, a carbon component, a reaction accelerator and water and having a water content of from 1 to 30 % by weight and a water mobility value of less than 0.01 to a contact treatment with an oxidizing gas under circumstances at 0 °C or higher, thereby regulating a temperature rise at 1 °C or higher within 10 minutes. So far as some change is caused in the reaction mixture by the contact treatment with an oxidizing gas, the iron powder is not always required to be oxidized. However, it is preferable that the iron powder is oxidized. In that case, it is preferable that the iron powder becomes an active iron powder.

The "active heat generating composition" as referred to herein is a heat generating composition corresponding to any one of the following (1) to (3).

(1) A heat generating composition prepared by contact treating a reaction mixture containing, as essential components, an iron powder, a carbon component, a reaction accelerator and water with an oxidizing gas, or by subjecting the oxidizing gas and the contact treated mixture to adjustment of the water content by the addition of water or a reaction accelerator aqueous solution.

(2) A heat generating composition prepared by contact treating a reaction mixture containing, as essential components, an iron powder, a carbon component, a reaction accelerator and water and having a water content of from 1 to 30 % by weight and a water mobility value of less than 0.01 with an oxidizing gas under circumstances at 0 °C or higher, thereby regulating a temperature rise at 1 °C or higher within 10 minutes, or by subjecting the oxidizing gas and the contact treated mixture to adjustment of the water content by the addition of water or a reaction accelerator aqueous solution.

(3) A heat generating composition containing, as essential components, an iron powder, a carbon component, a reaction accelerator and water, wherein an iron powder containing from 20 to 100 % of an active iron powder is used as the iron powder.

The "moldable heat generating composition" as referred to herein is a heat generating composition which contains, as essential components, an iron powder, a carbon component, a reaction accelerator and water but does not contain a flocculant aid, a flocculant, an agglomeration aid, a dry binder, a dry binding agent, a dry binding material, a sticky raw material, a thickener, an excipient, an alcohol, a crosslinking agent and a plasticizer, contains surplus water so as to have a water mobility value of from 0.01 to 20 and has moldability due to the surplus water as a connecting substance, in which the water in the heat generating composition does not function as a barrier layer, the heat generating composition being capable of causing an exothermic reaction upon contact with air.

The "perforation" as referred to in the invention includes one which is intermittently cut for improving flexural properties of the sectioned part and one which is intermittently cut such that cutting by hand is possible. Its degree is not limited but is determined depending upon the desire. The perforation may be provided in all sectioned parts or may be partially provided. The shape is not particularly limited, and examples thereof include a circle, an ellipse, a rectangle, a square, and a cut line (linear shape). For example, in the perforation which is intermittently cut such that cutting by hand is possible, a circular hole having an aperture of from $\phi$10 to 1,200 $\mu$m can be enumerated. The aperture of the hole is more preferably from $\phi$20 to 500 $\mu$m. When the aperture of the hole becomes $\phi$20 $\mu$m or less, cutting properties by hand may possibly be deteriorated due to an increase of the cutting strength of the film, or breakage or fray on the cut surface tends to be generated; and when the aperture of the hole is less than $\phi$10 $\mu$m, such a tendency especially becomes remarkable, and therefore, such is not preferable. On the other hand, when the aperture of the hole becomes $\phi$500 $\mu$m or more, shape destruction such as breakage may possibly be introduced due to a lowering of the cutting strength, and stability tends to be lowered due to a lowering of the workability or line aptitude at the time of production, oozing, or vaporization and volatilization; and when the aperture of the hole exceeds $\phi$1,200 $\mu$m, such a tendency especially becomes remarkable, and therefore, such is not preferable.

It is preferable that the holes are positioned lined up in the length and width. Furthermore, a shortest space between outer peripheries of the adjacent holes in the length and width is preferably from 10 to 2,000 $\mu$m, more preferably from 10 to 1,500 $\mu$m, further preferably from 20 to 1, 000 $\mu$m, still further preferably from 20 to 500 $\mu$m, and even further preferably from 20 to 200 $\mu$m. When the shortest space between outer peripheries of the adjacent holes in the length and width is less than 10 $\mu$m, shape destruction such as breakage may possibly be introduced due to a lowering of the cutting strength and a lowering of the workability or line aptitude at the time of production is found, and therefore, such is not preferable. On the other hand, when the shortest space between outer peripheries of the adjacent holes in the length and width exceeds 2,000 $\mu$m, cutting properties by hand may possibly be deteriorated due to an increase of the cutting strength of the film and breakage or fray on the cut surface tends to be generated, and therefore, such is not preferable. That is, the cutting properties by hand are remarkably improved by a balance between the aperture of the processed hole and the shortest space of outer peripheries of the adjacent holes in the length and width.

The hole may be a cut line, and its length may be a length corresponding to the aperture. A shortest space between ends of the adjacent cut lines in the length and width is corresponding to the shortest space between outer peripheries of the adjacent holes.

For example, an aperture of the hole of from $\phi$10 to 2, 000 $\mu$m is corresponding to a length of from 10 to 2,000 $\mu$m, and a shortest space between outer peripheries of the adjacent holes in the length and width of from 10 to 2,000 $\mu$m is corresponding to a shortest space between ends of the adjacent cut lines in the length and width of from 10 to 2,000 $\mu$m.

The fixing means is not limited so far as it has capability for fixing a thermal packaging body for joint surroundings or a material having an exothermic part to a prescribed part.

As the fixing means, an adhesive layer, a hook and eye, a hook and button, a hook and loop fastener such as Velcro, a magnet, a band, a string, and combination thereof can be arbitrarily used.

Incidentally, in the case of a band, fixing means for adjustment may be further constructed by a combination of a hook and loop fastener and an adhesive layer.

Here, the "hook and loop fastener" as referred to herein has a fastening function by a combination of a loop as a female fastener with a male fastener capable of fastening the female fastener thereto, which is known as trade names such as Magic Tape (a registered trademark), Magic Fastener (a registered trademark), Velcro Fastener, and Hook and Loop Tape. Examples of the material having a loop function include non-woven fabrics and woven fabrics of napped or hole-containing yarns. Such a material having a loop function (female fastener function) may be covered on the surface of a paddling forming the band, or the band may be constructed of such a material itself. Although the hook member which is the male fastener member is not particularly limited, examples thereof include hook members formed of a polyolefin based resin (for example, polyethylene and polypropylene), a polyamide, a polyester, etc. Although the shape of the hook is not particularly limited, a hook having a cross-sectional shape such as an I type, an inverted L type, an inverted J type, and a so-called mushroom type is preferable because it is easily hooked by the loop and does not give an extreme stimulus to the skin. Incidentally, the hook may be adhered to the entire area of a fastening tape, and only the hook may be used as a fastening tape while omitting a tape substrate.

The adhesive layer may contain at least one member selected from additional components consisting of a water retaining agent, a water absorptive polymer, a pH adjusting agent, a surfactant, an organosilicon compound, a hydrophobic polymer compound, a pyroelectric substance, an antioxidant, an aggregate, a fibrous material, a moisturizer, a functional substance, and a mixture thereof.

The adhesive of the invention is classified into a non-hydrophilic adhesive, a mixed adhesive, and a hydrophilic adhesive (for example, a gel).

The adhesive constituting the adhesive layer is not limited so far as it has an adhesive strength necessary for adhering to the skin or clothes. Adhesives of every form such as a solvent based adhesive, an aqueous adhesive, an emulsion type adhesive, a hot melt type adhesive, a reactive adhesive, a pressure-sensitive adhesive, a non-hydrophilic adhesive, and a hydrophilic adhesive are employable.

The adhesive layer includes one layer of a non-hydrophilic adhesive constituted of the non-hydrophilic adhesive and non-hydrophilic adhesive layers constituted of the non-hydrophilic adhesive.

It is to be noted that a material whose water absorption properties are improving by containing a water absorptive polymer or a water retaining agent in the non-hydrophilic adhesive layer is dealt as the non-hydrophilic adhesive layer.

A hot melt based adhesive may be provided between the hydrophilic adhesive layer and a substrate or a covering material. Furthermore, in the case where the hydrophilic adhesive is provided in a thermal packaging body for joint surroundings, there is no limitation. After seal treating a thermal packaging body for joint surroundings, a hydrophilic adhesive layer may be provided in the thermal packaging body for joint surroundings.

Furthermore, the adhesive layer may or may not have air permeability and may be properly selected depending upon the utility. With respect to the air permeability, the adhesive layer may be air-permeable as a whole. Examples thereof include an adhesive layer having air permeability as a whole of a region in which an adhesive is partially present and a portion where no adhesive is present is partially present.

In laminating an adhesive on an air-permeable substrate and/or a covering material in a stratiform state as it is, examples of a method for keeping its air permeability include a method in which an adhesive layer is partially laminated by printing or transferring an adhesive, thereby forming a non-laminated part as an air-permeable part; a method in which an adhesive is transferred in one direction while drawing a circle in a filament-like form or properly moved in the two-dimensional directions by transferring in a zigzag manner, whereby a space of the filament-like adhesive keeps air permeability or moisture permeability or the adhesive is foamed; and a method for forming a layer by a melt blow system. Examples of the adhesive which constitutes the non-hydrophilic adhesive layer include acrylic adhesives, polyvinyl acetate based adhesives (for example, vinyl acetate resin based emulsions and ethylene-vinyl acetate resin based holt melt adhesives), polyvinyl alcohol based adhesives, polyvinyl acetal based adhesives, vinyl chloride based adhesives, polyamide based adhesives, polyethylene based adhesives, cellulose based adhesives, chloroprene (neoprene) based adhesives, nitrile rubber based adhesives, polysulfide based adhesives, butyl rubber based adhesives, silicone rubber based adhesives, styrene based adhesives (for example, styrene based hot melt adhesives), rubber based adhesives, and silicone based adhesives. Of these, rubber based adhesives, acrylic adhesives, and adhesives containing a hot melt based polymer substance for the reasons that they are high in the adhesive strength, are cheap, are good in long-term stability, and are small in reduction of the adhesive strength even by providing heat.

In addition to the base polymer, if desired, the adhesive may be compounded with other components such as tackifiers (for example, petroleum resins represented by rosins, chroman-indene resins, hydrogenated petroleum resins, maleic anhydride-modified rosins, rosin derivatives, and C-5 based petroleum resins), phenol based tackifiers (especially, tackifiers having an aniline point of not higher than 50 °C; for example, terpene phenol based resins, rosin phenol based resins, and alkylphenol based resins), softeners (for example, coconut oil, castor oil, olive oil, camellia oil, and liquid paraffin), softeners, anti-aging agents, fillers, aggregates, adhesion adjusting agents, adhesion modifiers, coloring agents, anti-foaming agents, thickeners, and modifiers, thereby improving performance such as an improvement in adhesion to nylon-made clothes and mixed yarn clothes.

Examples of the hot melt based adhesive include known hot melt based adhesives imparted with adhesion. Specific examples thereof include styrene based adhesives made of, as a base polymer, an A-B-A type block copolymer (for example, SIS, SBS, SEBS, and SIPS), vinyl chloride based adhesives made of, as a base polymer, a vinyl chloride resin, polyester based adhesives made of, as a base polymer, a polyester, polyamide based adhesives made of, as a base polymer, a polyamide, acrylic adhesives made of, as a base polymer, an acrylic resin, polyolefin based adhesives made of, as a base polymer, a polyolefin (for example, polyethylene, super low density polyethylene, polypropylene, ethylene-$\alpha$-olefin copolymers, and ethylene-vinyl acetate copolymers), 1,2-polybutadiene based adhesives made of, as a base polymer, 1,2-polybutadiene, and polyurethane based adhesives made of, as a base polymer, polyurethane; adhesives made of a modified body of the foregoing adhesive whose adhesion is improved or whose stability is changed; and mixtures of two or more kinds of these adhesives. Adhesive layers constituted of a foamed adhesive and adhesive layers constituted of a crosslinked adhesive can also be employed.

The non-aromatic hot melt based adhesive is not limited so far as it is made of, as a base polymer, a hot melt based adhesive not containing an aromatic ring. Examples thereof include olefin based hot melt based adhesives and acrylic hot melt based adhesives. As the non-aromatic polymer which is the base polymer not containing an aromatic ring, there are enumerated polymers or copolymers of an olefin or a diene. Examples thereof include olefin polymers. The olefin polymer includes polymers or copolymers of ethylene or an $\alpha$-olefin. Also, polymers resulting from adding a diene (for example, butadiene and isoprene) as other monomer thereto may be employed.

The $\alpha$-olefin is not limited so far as it is a monomer having a double bond in the terminal thereof. Examples thereof include propylene, butene, heptane, hexene, and octene.

The "aromatic hot melt based adhesive" as referred to herein is a hot melt based adhesive whose base polymer contains an aromatic ring. Examples thereof include styrene based hot melt based adhesives represented by A-B-A type block copolymers.

In the foregoing A-B-A type block copolymers, the A block is a non-elastic polymer block made of a monovinyl substituted aromatic compound A such as styrene and methylstyrene; and the B block is an elastic polymer block made of a conjugated diene such as butadiene and isoprene. Specific examples thereof include a styrene-butadiene-styrene block copolymer (SBS), a styrene-isoprene-styrene block copolymer (SIS), and hydrogenated types thereof (for example, SEBS and SIPS), and mixtures thereof.

As a countermeasure for preventing a lowering of adhesive strength caused due to an increase of water of the non-hydrophilic adhesive layer, an adhesive layer obtained by further compounding a water absorptive polymer in the non-hydrophilic adhesive can be used.

The hydrophilic adhesive which constitutes the hydrophilic adhesive layer is not particularly limited so far as it contains a hydrophilic polymer or a water-soluble polymer as the major component, has adhesion and is hydrophilic as an adhesive. Examples of the constitutional components of the hydrophilic adhesive include hydrophilic polymers (for example, polyacrylic acid), water-soluble polymers (for example, poly(sodium acrylate) and polyvinylpyrrolidone), crosslinking agents (for example, dry aluminum hydroxide and meta-silicic acid aluminic acid metal salts), softeners (for example, glycerin and propylene glycol), higher hydrocarbons (for example, soft liquid paraffin and polybutene), primary alcohol fatty acid esters (for example, isopropyl myristate), silicon-containing compounds (for example, silicone oil), fatty acid glycerin esters (for example monoglycerides), oily components (for example, vegetable oils such as olive oil), antiseptics (for example, methyl p-hydroxybenzoate and propyl p-hydroxybenzoate), solubilizing agents (for example, N-methyl-2-pyrrolidone), thickeners (for example, carboxymethyl cellulose), surfactants (for example, polyoxyethylene hardened castor oil and sorbitan fatty acid esters), hydroxycarboxylic acid (for example, tartaric acid), excipients (for example, light silicic anhydride, water absorptive polymers, and kaolin), moisturizers (for example, D-sorbitol), stabilizers (for example, sodium edetate, p-hydroxybenzoic acid esters, and tartaric acid), crosslinking type water absorptive polymers, boron compounds (for example, boric acid), and water. They may be used as an arbitrary combination.

A temporary adhering seal part is formed via a sticky layer. An adhesive which constitutes the sticky layer is a layer formed of a polymer composition which is tacky at the normal temperature and is not limited so far as it can be heat sealed after temporary adhesion.

Furthermore, the foregoing adhesives of the sticky layer can be used as the adhesive which constitutes the sticky layer as used for temporary adhesion. Of these, non-hydrophilic adhesives are preferable. With respect to the adhesive constituting the adhesive layer, it is preferable that the adhesive is well compatible with a heat seal material constituting a heat seal and that a melting point of the base polymer of the adhesive is not higher than a melting point of the heat seal material. Hot melt based adhesives are especially preferable for hot melt based bonding agents. Furthermore, in the case where the heat seal material is an olefin based raw material, preferred examples thereof include olefin based adhesives.

A bonding layer for fixing the air permeability adjusting material is constituted of a bonding agent or an adhesive which is usually used. In particular, an adhesive is useful, and the foregoing adhesives for constituting the adhesive layer can be used.

Furthermore, a method for providing a bonding layer is not limited so far as the air permeability adjusting material can be fixed. The bonding layer may be entirely provided or partially or intermittently provided. Examples of its shape include various shapes such as a network-like shape, a stripe-like shape, a dot-like shape, and strip-like shape.

Furthermore, in the case where an adhesive layer is employed as the hydrophilic adhesive layer, if there is a difference in a water retaining force between the hydrophilic adhesive layer and the heat generating composition molded body, transfer of water occurs via a packaging material present therebetween such as a substrate, thereby causing in-conveniences against the both. In particular, the transfer of water occurs during the storage. In order to prevent this, it is preferable that the packaging material present therebetween at least has a moisture permeability of not more than 2 $g/m^2/day$ in terms of a moisture permeability according to the Lyssy method. By using this, in the case where the heat generating body is accommodated in an outer bag as an air-impermeable accommodating bag and stored, the transfer of water can be prevented.

In the case where a hydrophilic adhesive layer is used as the adhesive layer, the moisture permeability of a moisture-proof packaging material provided between the heat generating composition molded body and the hydrophilic adhesive layer is not limited so far as the transfer of water can be prevented within the range where the exothermic performance is not affected. The moisture permeability according to the Lyssy method is usually not more than 2 $g/m^2/day$, preferably not more than 1.0 $g/m^2/day$, more preferably not more than 0.5 $g/m^2/day$, and further preferably from 0.01 to 0.5 $g/m^2/day$. These values are a value under a condition under an atmospheric pressure at 40 °C and 90 % RH. Incidentally, the moisture-proof packaging material can be used as a substrate or a covering material and may be laminated singly on a substrate, a covering material, or the like.

The moisture-proof packaging material is not limited so far as the transfer of water between the heat generating composition molded body and the hydrophilic adhesive layer can be prevented. Examples thereof include metal vapor deposited films, vapor deposited films of a metal oxide, metal foil-laminated films, EVOH (ethylene/vinyl alcohol copolymer or ethylene/vinyl acetate copolymer saponified product) based films, biaxially stretched polyvinyl alcohol films, polyvinylidene chloride coated films, polyvinylidene chloride coated films obtained by coating polyvinylidene chloride on a substrate film (for example, polypropylene), metal foils such as an aluminum foil, air-impermeable packaging materials obtained by vapor depositing or sputtering a metal (for example, aluminum) on a polyester film substrate, and packaging laminates using a transparent barrier film of a structure in which silicon oxide or aluminum oxide is provided on a flexible plastic substrate. The air-impermeable packaging materials which are used in the outer bag, etc. can also be used.

Furthermore, packaging materials such as moisture-proof packaging materials as described in JP-A-2002-200108, the disclosures of which can be incorporated herein by reference, can be used.

In the case of using a water-containing hydrophilic adhesive (for example, a gel) in the adhesive layer, in order to adjust the moisture equilibrium between the heat generating composition and the adhesive layer, the content of a reaction accelerator (for example, sodium chloride) or a substance having a water holding power (for example, a water absorptive polymer) in the heat generating composition may be adjusted within the range of from 10 to 40 % by weight, preferably from 15 to 40 % by weight, and more preferably from 15 to 30 % by weight based on the heat generating composition.

Furthermore, as the adhesive having good moisture permeability and low stimulation to the skin, water-containing adhesives (for example, hydrophilic adhesives and gels) as described in JP-A-10-265373 and JP-A-9-87173, adhesives which can be subjected to hot melt coating as described in JP-A-6-145050 and JP-A-6-199660, and rubber based adhesives as described JP-A-10-279466 and JP-A-10-182408, the disclosures of which are totally incorporated herein by reference, are useful.

The functional substance which is contained in the adhesive layer is not limited so far as it is a substance having any function. There can be enumerated at least one member selected from aromatic compounds, vegetable extracts, crude drugs, perfumes, slimming agents, analgesics, blood circulation promoters, swelling improvers, antibacterial agents, sterilizers, mold inhibitors, odor eaters, deodorants, percutaneously absorptive drugs, fat-splitting components, minus ion generators, far infrared ray radiants, magnetic bodies, fomentations, cosmetics, bamboo vinegar, and wood vinegar. Specific examples thereof include aromatic compounds (for example, menthol and benzaldehyde), vegetable extracts (for example, mugwort extract), crude drugs (for example, moxa), perfumes (for example, lavender and rosemary), slimming agents (for example, aminophylline and tea extract), analgesic drugs (for example, indomethacin and dl-camphor), blood circulation promoters (for example, acidic mucopolysaccharide and chamomile), swelling improvers (for example, horse chestnut extract and flavone derivatives), fomentations (for example, aqueous boric acid, physiological saline, and aqueous alcohols), fat-splitting components (for example, jujube extract, caffeine, and tonalin), cosmetics (for example, aloe extracts, vitamin preparations, hormone preparations, anti-histamines, and amino acids), antibacterial agents and sterilizers (for example, carbolic acid derivatives, boric acid, iodine preparations, invert soaps, salicylic acid based substances, sulfur, and antibiotics), and mold inhibitors.

The percutaneously absorptive drug is not particularly limited so far as it has percutaneous absorption. Examples thereof include corticosteroids, anti-inflammatory drugs, hypertension drugs, anesthetics, hypnotic sedatives, tranquillizers, antibacterial substances, antifungal substances, skin stimulants, inflammation inhibitors, anti-epileptics, analgesics, antipyretics, anesthetics, mold inhibitors, antimicrobial antibiotics, vitamins, antiviral agents, swelling improvers, diuretics, antihypertensives, coronary vasodilators, anti-tussive expectorants, slimming agents, anti-histamines, antiarrhythmic agents, cardiotonics, adrenocortical hormones, blood circulation promoters, local anesthetics, fat-splitting components, and mixtures thereof. However, it should not be construed that the invention is limited thereto. These drugs are used singly or in admixture of two or more kinds thereof as the need arises.

The content of the percutaneously absorptive drug is not particularly limited so far as it falls within the range where the effect of a medicine can be expected. However, from the viewpoints of adhesive strength as well as pharmacological effect and economy, the content of the functional substance is preferably from 0.01 to 25 parts by weight, and more preferably from 0.5 to 15 parts by weight based on 100 parts by weight of the adhesive.

Furthermore, a method for providing the adhesive layer is not limited so far as a thermal packaging body for joint surroundings can be fixed. The adhesive layer may be entirely provided or partially or intermittently provided. Examples of its shape include various shapes such as a network-like shape, a stripe-like shape, a dot-like shape, and strip-like shape.

The term "substantially planar" as referred to in the invention means a planar surface not having an accommodating concave such as an accommodating pocket, an accommodating section, and an accommodating zone as provided in advance for the purpose of accommodating the heat generating composition. Accordingly, irregularities which do not intentionally accommodate the heat generating composition may be present.

The "pocket" as referred to in the invention is an accommodating pocket which is provided in advance for the purpose of accommodating the heat generating composition and is a pocket as described in JP-T-2001-507593. Since irregularities which are not used for intentionally accommodating the heat generating composition molded body are not the pocket, even when such irregularities are present on a substrate, it is to be noted that such a substrate is defined as a substantially planar substrate.

The "accommodating section" as referred to herein is an accommodating section for accommodation as provided in advance on the packaging material for the purpose of accommodating the heat generating composition and is an accommodating section as described in Japanese Patent No. 3,161,605 and JP-T-11-508314. Since irregularities which are not used for intentionally accommodating the heat generating composition molded body are not the accommodating section, even when such irregularities are present on a substrate, it is to be noted that such a substrate is defined as a substantially planar substrate.

The "accommodating zone" as referred to herein is an accommodating zone for accommodation as provided in advance on the packaging material for the purpose of accommodating the heat generating composition and is an accommodating zone as described in Japanese Patent No. 3,161,605 and JP-T-11-508314. Since irregularities which are not used for intentionally accommodating the heat generating composition molded body are not the accommodating zone, even when such irregularities are present on a substrate, it is to be noted that such a substrate is defined as a substantially planar substrate.

Furthermore, so far as the reaction characteristics and exothermic characteristics are not affected, the heat generating composition having a water mobility value of less than 0.01 may contain a flocculant aid, a flocculant, an agglomeration aid, a dry binder, a dry binding agent, a dry binding material, a sticky raw material, a thickener, an excipient, or a water-soluble polymer in an amount ranging from 0.01 to 3 parts by weight.

The "flocculant aid" as referred to herein is a flocculant aid as described in Japanese Patent No. 3161605 (JP-T-11-508314) such as gelatin, natural gum, and corn syrup.

The "flocculant" as referred to herein is a flocculant as described in JP-T-2002-514104 such as corn syrup and maltitol syrup.

The "agglomeration aid" as referred to herein is an agglomeration aid as described in JP-T-2001-507593 such as corn syrup.

The "dry binder" as referred to herein is a dry binder as described in JP-T-2002-514104 such as microcrystalline cellulose, maltodextrin, and mixtures thereof.

The "dry binding agent" as referred to herein is a dry binding agent as described in JP-T-2001-507593 such as maltodextrin and sprayed lactose.

The "dry binding material" as referred to herein is a dry binding material as described in JP-T-11-508314 such as microcrystalline cellulose, maltodextrin, and mixtures thereof.

The "sticky raw material" or the "binder" as referred to herein is a sticky raw material or binder as described in JP-A-4-293989 such as water glass, polyvinyl alcohol (PVA), and carboxymethyl cellulose (CMC).

The "thickener" as referred to herein is a thickener as described in JP-A-6-343658 such as corn starch and potato starch.

The "excipient" as referred to herein is an excipient as described in JP-A-7-194641 such as $\alpha$-starch and sodium alginate.

As the "water-soluble polymer" as referred to herein, the water-soluble polymer in the adhesive layer can be used.

The term "non-flexible" means that even when a bending test of the subjective heat generating body as defined in JIS B2403 is carried out, a crack or the like is not observed at all and that when bent at 90°, the heat generating body is broken and does not restore to the original shape, an aspect of which is opposite to that of the term "flexible" which means that even when bent at 90°, the heat generating body is not broken.

In the invention, as a heat seal material constituting a heat seal layer, a single raw material may be used, or a composite raw material having a heat seal layer may be used. The heat seal material is not limited so far as at least a part thereof can be welded upon heating. Examples thereof include hot melt based resins such as polyolefins (for example, polyethylene and polypropylene) or olefin copolymer resins, ethylene based hot melt resins (for example, ethylene-vinyl acetate copolymer resins and ethylene-acrylic acid ester copolymer resins (for example, ethylene-isobutyl acrylate copolymer resins)), polyamide based hot melt resins, butyral based hot melt resins, polyester based hot melt resins, polyamide based hot melt resins, polyester based hot melt resins, polymethyl methacrylate based hot melt resins, polyvinyl ether based hot melt resins, polyurethane based hot melt resins, polycarbonate based hot melt resins, such as polyvinyl acetate, and vinyl chloride-vinyl acetate copolymers; and films or sheets thereof. Furthermore, in these hot melt based resins or films or sheets thereof, ones having various additives (for example, an antioxidant) compounded therein can be used. In particular, low density polyethylene and polyethylene obtained by using a metallocene catalyst are useful.

In the case of interposing a heat generating composition molded body between a substrate and a covering material, the "temporary adhesion" as referred to in the invention means weak pressure-sensitive bonding or adhesion for the purpose of holding the accommodated heat generating composition molded body until at least the substrate and the covering material are adhered to each other via a sticky layer made of an adhesive and heat sealed.

Furthermore, the "deadhesion" as referred to herein means that in the temporary adhering seal part after heat seal, the heat generating composition in a non-heat sealed region is transferred to the foregoing region, thereby releasing the temporary adhesion.

The temporary adhering seal part is formed via a sticky layer. An adhesive constituting the sticky layer is not limited so far as it is a layer formed of a polymer composition which is tacky at the normal temperature and can be heat sealed after the temporary adhesion.

Furthermore, although the adhesive of the foregoing adhesive layer can be used as the adhesive constituting the sticky layer to be used for the temporary adhesion, a non-hydrophilic adhesive is preferable. As the adhesive constituting the sticky layer, one which is well compatible with the heat seal material constituting the heat seal is preferable, and a melting point of a base polymer of the adhesive is preferably not higher than a melting point of the heat seal material. In particular, hot melt based adhesives are preferable. Furthermore, in the case where the heat seal material is made of an olefin based raw material, preferred examples of the adhesive include olefin based adhesives.

Incidentally, a method for providing a sticky layer for the temporary adhesion is not limited. The sticky layer may be entirely provided or partially or intermittently provided. Examples of its shape include various shapes such as a network-like shape, a stripe-like shape, a dot-like shape, and strip-like shape.

A capacity of the sectional exothermic part or a volume of the wettable heat generating composition compressed body is usually from 0.015 to 500 cm$^3$, preferably from 0.04 to 30 cm$^3$, more preferably from 0.1 to 30 cm$^3$, further preferably from 1 to 30 cm$^3$, and still further preferably from 3 to 20 cm$^3$.

In the sectional exothermic part, when the sectional exothermic part which is an accommodating region of the heat generating composition is filled with the heat generating composition molded body, a capacity ratio of the volume of the heat generating composition molded body which is an occupying region of the heat generating composition molded body to the capacity of the sectional exothermic part which is an accommodating region of the heat generating composition is usually from 0.6 to 1, preferably from 0.7 to 1, more preferably from 0.8 to 1, and further preferably from 0.9 to 1.0.

Furthermore, a width of the sectioned part which is a space between the sectional exothermic parts is not limited so far as sectioning can be achieved. It is usually from 0.1 to 50 mm, preferably from 0.3 to 50 mm, more preferably from 0.3 to 50 mm, further preferably from 0.3 to 40 mm, still further preferably from 0.5 to 30 mm, even further preferably from 1.0 to 20 mm, and even still further preferably from 3 to 10 mm.

Furthermore, the "capacity of the sectional exothermic part" as referred to herein means an internal capacity of the sectional exothermic part having a heat generating composition molded body accommodated therein.

Furthermore, a region where the air permeability is higher than that in the covering part for covering the heat generating composition molded body may be provided in a local region of the air-permeable adjusting material, thereby keeping the air permeability of other region lower than that on the air-permeable surface of the sectional exothermic part. In this way, it is possible to control an air passage for air, etc.

The fixing region between the air permeability adjusting material and the exothermic part is not limited so far as the both can be fixed and air can go in and out from at least the periphery of the sectional exothermic part. However, the following can be enumerated.

1) The fixing region is fixed in the both ends of the exothermic part or heat generating body.
2) A space is provided entirely in a substantially central part of the exothermic part, and other exothermic part region is defined as the fixing region.
3) A substantially top part of each sectional exothermic part and a substantially central part of each sectioned part are defined as the fixing region.

Here, as the air permeability adjusting material, any material can be used so far as it is provided with a space which communicates with the outside in the surroundings of the sectional exothermic part. Examples of an air permeability adjusting material having a bonding layer and utilizing a plastic film include PE/adhesive, PP/adhesive, polyester/adhesive, PE/non-woven fabric/air-permeable adhesive, PE/non-woven fabric/PE/adhesive, PE/PET/M/PE/non-woven fabric/air-permeable adhesive, PE/heat seal material, PE/non-woven fabric/heat seal material, PE/non-woven fabric/PE/ heat seal material, and PE/polyester/M/PE/non-woven fabric/heat seal material. Here, M represents a metal (for example, aluminum and silver), a semiconductor (for example, silicon oxide, silicon oxynitride, silicon nitride, and aluminum oxide), or a metal oxide, oxynitride or nitride. Furthermore, a portion for placing fixing means such as an adhesive layer and a heat sealing agent layer is not limited, and whether it is provided partially or entirely may be properly determined depending upon the intended purpose.

The bonding layer for fixing the air permeability adjusting material is not limited so far as the air permeability adjusting material can be fixed to the heat generating body and is constituted of a usually used bonding agent or adhesive. In particular, an adhesive is useful, and the adhesive constituting the foregoing adhesive layer can be used.

Furthermore, a method for providing the bonding layer is not limited so far as the air permeability adjusting material can be fixed. The bonding layer may be entirely provided or partially or intermittently provided. Examples of its shape include various shapes such as a network-like shape, a stripe-like shape, a dot-like shape, and strip-like shape. Its thickness is not particularly limited but is in the range of from 5 to 1, 000 $\mu$m, preferably from 10 to 500 $\mu$m, and more preferably from 15 to 250 $\mu$m. When the thickness of the bonding layer is less than 5 $\mu$m, a desired adhesive strength may not be possibly obtained. On the other hand, when it exceeds 1,000 $\mu$m, not only it becomes bulky and becomes worse in feeling for use, but also it becomes worse in economy, and therefore, such is not preferable.

The heat generating body of the invention is able to give various shapes, thicknesses and temperature zones and therefore, can be used for various utilities such as use for a joint, facial esthetic use, use for eyes, slimming use, use for heating or warming a dripping solution, use for a wet compress pack, use for a medical body warmer, use for a neck, use for a waist, use for a mask, use for a glove, use for hemorrhage, use for relaxation of symptoms such as shoulder pain, muscular pain, and menstrual pain, use for a cushion, use for heating or warming a human body during the operation, use for a thermal sheet, use for thermally volatilizing an aroma, use for an abdomen, insecticidal use by thermal volatilization, and use for treating cancer in addition to common warming of a human body. In addition, the heat generating body of the invention can be used for heating or warming machines, pets, etc.

For example, in the case of using for relaxation of symptoms, the heat generating body of the invention is applied directly in a necessary site of the body or indirectly via a cloth, etc. Furthermore, in the case of using for heating or warming a human body during the operation, a method for using the heat generating body of the invention includes the following methods.

(1) The heat generating body is directly applied to a body requiring heating or warming.
(2) The heat generating body is fixed on a covering, etc. and covered on the body.
(3) The heat generating body is fixed on a cushion to be placed beneath the body, etc.
(4) The heat generating body is used as a covering or a cushion which is a product having the heat generating body provided therein in advance.

Incidentally, examples of the pain of muscles or bones include acute muscle pain, acute bone pain, acute reference pain, previous muscle pain, previous bone pain, chronic reference pain, and join pain of knee, elbow, etc.

The holding time is not limited but is preferably from 20 seconds to 24 hours, more preferably from one hour to 24 hours, and further preferably from 8 hours to 24 hours.

The holding temperature is preferably from 30 to 50 °C, more preferably from 32 to 50 °C, further preferably from 32 to 43 °C, still further preferably from 32 to 41 °C, and even further preferably from 32 to 39 °C.

The invention will be described below in detail with reference to the following Examples.

[Brief Description of the Drawings]

[Fig. 1] is a graph showing exothermic characteristics of the heat generating composition compressed bodies as prepared in Example 1 and Comparative Examples 1 to 3.
[Fig. 2] is a plan view of a filter paper for the measurement of water mobility value in the invention.

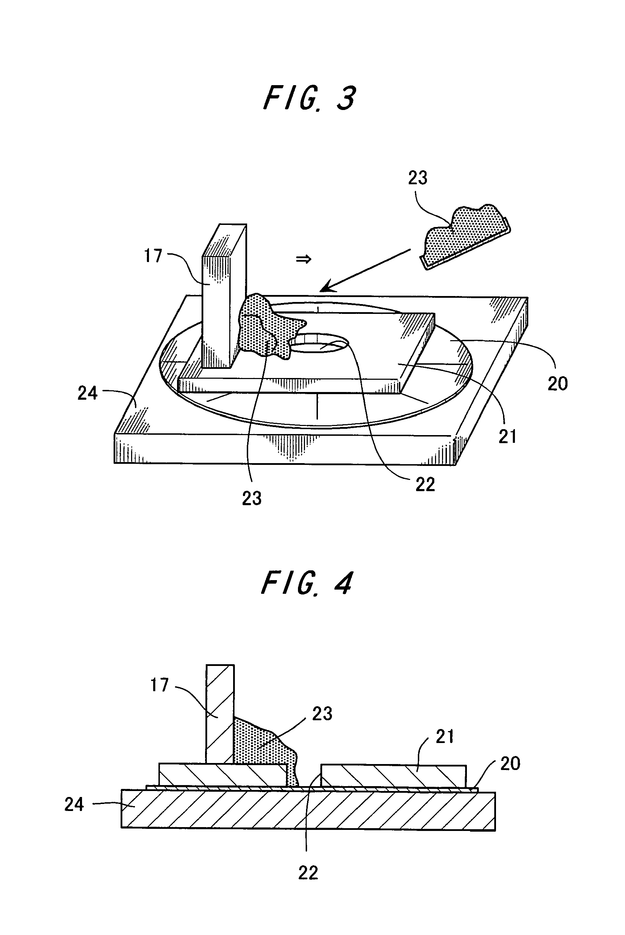

[Fig. 3] is an oblique view for explaining the measurement of water mobility value in the invention.
[Fig. 4] is a cross-sectional view for explaining the measurement of water mobility value in the invention.
[Fig. 5] is a cross-sectional view for explaining the measurement of water mobility value in the invention.
[Fig. 6] is a plan view of a filter paper after carrying out the measurement of water mobility value in the invention.

[Description of Reference Numerals and Signs]

17:  Pushing plate
18:  Flat plate
19:  Non-water absorptive film (polyethylene film, etc.)
20:  Filter paper in which eight lines are drawn radiating from the central point with an interval of 45°
21:  Die plate
22:  Hole
23:  Sample
24:  Stainless steel plate
25:  Distance to the oozed-out locus of water or aqueous solution
26:  Position corresponding to a hollow cylindrical hole on filter paper (circumferential part)

[Examples]

(Example 1)

A batchwise stirring tank consisting of a mixer equipped with a rotary blade of a blade shape of ventilation fan was used as an oxidizing gas contact treatment device, and air was used as an oxidizing gas. A heat generating mixture consisting of 59.5 parts by weight of an iron powder (particle size: not more than 300 $\mu$m), 5.95 parts by weight of active carbon (particle size: not more than 300 $\mu$m), 3.5 parts by weight of a water absorptive polymer (particle size: not more than 300 $\mu$m), and 5 parts by weight of 11 % salt water and having a water mobility value of less than 0.01 was charged in the oxidizing gas contact treatment device. Next, in the state that the upper portion of the contact treatment device vessel was opened to air, the reaction mixture was subjected to self heat generation with stirring under circumstances at 20 °C. After 30 seconds, at a point of time when a temperature rise of the reaction mixture reached 10 °C, 11 % salt water was mixed in the reaction mixture to obtain a moldable heat generating composition having a water mobility value of 8. Next, a cavity-containing trimming die of 3 mm in thickness×80 mm in width×110 mm in length was placed on a substrate made of a 60 $\mu$m-thick polyethylene film; the moldable heat generating composition was filled in the cavity by pushing molding; a pushing part-provided compression plate having a height of 1 mm was then covered thereon such that the pushing part was coincident with the cavity; and a pressure was applied, thereby molding a wettable heat generating composition compressed body having a thickness of 2 mm. The compression plate and the die were removed to obtain a wettable heat generating composition compressed body having a thickness of 2 mm as laminated on the substrate. The wettable heat generating composition compressed body was non-flexible.

(Comparative Example 1)

A heat generating composition having a water mobility value of 8.0 was obtained by using the blending of Example 1 in the same manner as in Example 1, except that the oxidizing gas contact treatment was not carried out. By using this heat generating compound, a heat generating composition compressed body was obtained in the same manner as in Example 1.

(Comparative Example 2)

0.1 parts by weight of glycerin or phosphorus was added to the blending of Example 1 prior to the oxidizing gas contact treatment, and 11 % salt water was further added, followed by stirring and mixing to obtain a heat generating composition having a water mobility value of 8.0. This heat generating composition was charged in a cavity-containing trimming die of 3 mm in thickness×80 mm in width×110 mm in length, a pushing part-provided compression plate having a height of 3 mm was then covered thereon such that the pushing part was coincident with the cavity, and a pressure of 7, 540 kg/cm$^2$ was applied for 10 seconds, thereby preparing a heat generating composition compressed body.

(Comparative Example 3)

A heat generating composition compressed body was obtained in the same manner as in Example 1 by using a heat generating composition of the blending of Comparative Example 2, except for using CMC in place of the glycerin.
A sensor was installed on the substrate surface beneath the substrate in the substantially central part of each of the heat generating composition compressed bodies as prepared in Example 1 and Comparative Examples 1 to 3, and an exothermic rising test of heat generating compressed body was carried out. The measurement results are shown in Fig. 1. As shown in Fig. 1, in the case of Example 1, the temperature reached 50 °C after 3 minutes and 73 °C after 10 minutes, respectively. In the case of Comparative Example 1, the temperature reached 41 °C after 3 minutes and 45 °C after 10 minutes, respectively. In the case of Comparative Example 2, the temperature reached 38 °C after 3 minutes and 43 °C after 10 minutes, respectively. In the case of Comparative Example 3, the temperature reached 40 °C after 3 minutes and 45 °C after 10 minutes, respectively. In the light of the above, it was noted that the heat generating body of Example 1 has much more excellent characteristics in view of the practical use.

(Example 2)

A batchwise stirring tank consisting of a mixer equipped with a rotary blade of a blade shape of ventilation fan was used as an oxidizing gas contact treatment device, and air was used as an oxidizing gas. A heat generating mixture consisting of 100 parts by weight of an iron powder (particle size: not more than 300 $\mu$m), 5.0 parts by weight of active carbon (particle size: not more than 300 $\mu$m), 3.5 parts by weight of a water absorptive polymer (particle size: not more than 300 $\mu$m), 2.0 parts by weight of a wood meal, and 5 parts by weight of 11 % salt water and having a water mobility value of less than 0.01 was charged in the contact treatment device vessel. Next, in the state that the upper portion of the contact treatment device vessel was opened to air, the heat generating mixture was subjected to self heat generation with stirring under circumstances at 30 °C and contact treated with an oxidizing gas at a maximum exothermic temperature of 67 °C until the exothermic temperature reached 35 °C, thereby obtaining an oxidizing gas contact treated heat generating mixture. 11 % salt water was mixed in this heat generating mixture to obtain a moldable heat generating composition having a water mobility value of 8. A cavity-containing trimming die of 3 mm in thickness×80 mm in width×110 mm in length was placed on a substrate made of a 60 $\mu$m-thick separator-provided polyethylene film provided with an acrylic adhesive layer; the moldable heat generating composition was filled in the cavity by pushing molding; a pushing part-provided compression plate having a height of 1 mm was then covered thereon such that the pushing part was coincident with the cavity; and a pressure was applied, thereby molding a wettable heat generating composition compressed body having a thickness of 2 mm. The compression plate and the die were removed to obtain a wettable heat generating composition compressed body having a thickness of 2 mm as laminated on the substrate. This wettable heat generating composition compressed body was non-flexible. A covering material made of a non-woven fabric-provided polyethylene-made porous film was covered thereon, the periphery of the wettable heat generating composition compressed body was heat sealed, and the outer circumferential part was cut while retaining a seal width of 8 mm, thereby obtaining a heat generating body. Next, the heat generating body was sealed in an outer bag which is an air-impermeable accommodating bag. Incidentally, the air permeability of the covering material was 600 g/m$^2$/24 hr in terms of a moisture permeability by the Lyssy method. After 24 hours, the heat generating body was taken out from the outer bag and subjected to an exothermic test by the body. As a result, it was felt warm after 3 minutes, and thereafter, the warmth was continued for 10 hours or more.

(Example 3)

A batchwise stirring tank consisting of a mixer equipped with a rotary blade of a blade shape of ventilation fan was used as an oxidizing gas contact treatment device, and air was used as an oxidizing gas. A heat generating mixture consisting of 100 parts by weight of a reduced iron powder (particle size: not more than 300 $\mu$m), 25 parts by weight of active carbon (particle size: not more than 300 $\mu$m), 3 parts by weight of a water absorptive polymer (particle size: not more than 300 $\mu$m), 0.5 parts by weight of calcium hydroxide, 0.7 parts by weight of sodium sulfite, and 5 parts by weight of 11 % salt water and having a water mobility value of less than 0.01 was charged in the contact treatment device vessel. Next, in the state that the upper portion of the contact treatment device vessel was opened to air, the heat generating mixture was subjected to self heat generation with stirring under circumstances at 30 °C and contact treated with an oxidizing gas at a maximum exothermic temperature of 67 °C until the exothermic temperature reached 35 °C, thereby obtaining an oxidizing gas contact treated heat generating mixture. 11 % salt water was mixed in the oxidizing gas contact treated heat generating mixture to obtain a moldable heat generating composition having a water mobility value of 7. Next, a substrate made of a laminate of a polyethylene film and a polypropylene-made non-woven fabric was placed on a stainless steel plate such that the side of the non-woven fabric was brought into contact with the stainless steel plate; a trimming die having a cavity of 10 mm in diameter×3 mm in height was subsequently placed on the side of the polyethylene film of the substrate; the moldable active heat generating composition was further placed on the trimming die; and the moldable active heat generating composition was filled into the cavity while leveling by using a leveling plate. Next, a compression plate having a convex having a height of 1 mm and capable of closely coming in the cavity in the central part thereof was placed such that the convex came in the cavity. The moldable active heat generating composition which had come in the cavity of die interposed between the stainless steel plate and the compression plate was compressed by passing through rolls, and the die was then removed to obtain a heat generating composition compressed body of 2 mm in thickness× 10 mm in diameter. Next, an air-permeable covering material made of a laminate of a polyethylene-made porous film and a nylon-made non-woven fabric was covered on the heat generating composition compressed body such that the side of the porous film was brought into contact with the heat generating composition compressed body, and the periphery of the heat generating composition compressed body was then heat sealed in a seal width of 8 mm, thereby obtaining a heat generating body having a diameter of 28 mm in terms of an outer dimension. This heat generating body was sealed and accommodated in an outer bag and allowed to stand at room temperature for 24 hours. Incidentally, the air permeability of the air-permeable covering material 6 was 400 g/m²/ 24 hr in terms of a moisture permeability by the Lyssy method. Collapsed pieces of the heat generating composition molded body were not generated in the surroundings of the heat generating composition molded body, heat sealing could be surely achieved, and cutting in seal was not generated. After 24 hours, the heat generating body was taken out from the outer bag and subjected to an exothermic test. As a result, the temperature reached 55 °C within 3 minutes.

(Example 4)

By using the substrate and the covering material of Example 2, the heat generating composition compressed bodies of 2 mm in thickness×10 mm in diameter of Example 4 were arranged fives in the length and three in the width, respectively at intervals of 5 mm; the periphery of each of the heat generating composition compressed bodies was heat sealed in the same manner as in Example 1; and the outer circumference of the heat generating body was further heat sealed, thereby obtaining a substantially rectangular heat generating body in which a 4-mm portion between the heat generating composition compressed bodies and an 8-mm portion of the outer circumference of the heat generating body were heat sealed. This heat generating body was sealed and accommodated in an outer bag and allowed to stand at room temperature for 24 hours. Incidentally, the air permeability of the air-permeable covering material 6 was 400 g/m²/ 24 hr in terms of a moisture permeability by the Lyssy method. Collapsed pieces of the heat generating composition molded body were not generated in the surroundings of the heat generating composition molded body, heat sealing could be surely achieved, and cutting in seal was not generated. After 24 hours, the heat generating body was taken out from the outer bag, stuck to the outside of underwear and subjected to an exothermic test by the body to which the heat generating body had been stuck on the abdominal region. As a result, it was felt warm after 3 minutes, and the proper temperature was continued for 8 hours or more. The heat generating body was convenient in handling and well followed and adapted with the curved face of the abdominal region.

(Example 5)

By using the moldable heat generating composition of Example 3, a wettable heat generating composition compressed body of 2 mm in thickness×10 mm in diameter was obtained in the same manner as in Example 3. The wettable heat generating composition compressed body was charged in an accommodating section made of a sheet of an EVA layer and a polypropylene layer and vacuum molded in a disc shape; a sheet made of an EVA layer and a polypropylene layer was further covered thereon such that the EVA layers were superimposed each other; and these two sheets were heat sealed to seal the wettable heat generating composition compressed body in an accommodating section between these sheets, thereby forming a pocket heat generating body. Next, an LDPE film was perforated, and the pocket heat generating body was sealed in an outer bag which is an air-impermeable accommodating body. After 24 hours, the pocket heat generating body was taken out from the outer bag and allowed to stand in air. As a result, the pocket heat generating body soon started to generate heat. The polypropylene non-woven fabric/LDPE sheet was perforated, and an oxygen-diffusible permeability of about 1.7 cc/min/5 cm² (at 21 °C and one atmosphere) was imparted. Furthermore, the pocket heat generating body had a height of about 2.1 mm and a diameter of about 11 mm, and a ratio of the volume of the exothermic part to the volume of the wettable heat generating composition compressed body was about 0.81. If a plural number of this pocket heat generating body is fixed on a non-woven fabric by an adhesive or the like and a fixing measure such as an adhesive layer is provided, the pocket heat generating body can be used for body wearing implements and the like likewise the heat generating body of Example 5.

**Claims**

**1.** A wettable heat generating composition compressed body resulting from compression of a moldable heat generating composition capable of causing an exothermic reaction upon contact with air, **characterized in that**:

1) the heat generating composition is one prepared by subjecting a reaction mixture containing, as essential components, an iron powder, a carbon component, a reaction accelerator, a crosslinking type water absorptive polymer and water but not containing a flocculant aid, a flocculant, an agglomeration aid, a dry binder, a dry binding agent, a dry binding material, a sticky raw material, a thickener, an excipient, an alcohol, a crosslinking agent and a plasticizer and having a water content of from 1 to 20 % by weight and a water mobility value of less than 0.01 to a contact treatment with an oxidizing gas under circumstances at 0 °C or higher, regulating a temperature rise of the reaction mixture at 1 °C or higher within 10 minutes, and adjusting the water content so as to contain surplus water having a water mobility value of from 0.01 to 20 and has moldability due to the surplus water as a connecting substance, in which the water in the heat generating composition does not function as a barrier layer; that

2) the moldable heat generating composition is molded by in-mold compression by filling in a mold cavity of a mold cavity-provided die to a depth substantially the same as the depth of the mold cavity and compressing to a thickness of from 50 to 99.5 % against the depth of the mold cavity; and that

3) the compressed body is non-flexible and has shape holding properties.

**2.** The wettable heat generating composition compressed body according to claim 1, **characterized in that** the heat generating composition contains at least one member selected from additional components consisting of a water retaining agent, a pH adjusting agent, a hydrogen formatio inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof.

**3.** The wettable heat generating composition compressed body as according to claim 1, **characterized in that** the iron powder comprising particles, a surface of each of which is at least partially covered with an iron oxide film; that the iron oxide film has a thickness of 3 nm or more; and that the active iron powder particles having a region of an oxygen-free iron component in at least one region selected from a central region of the iron powder particles and a region beneath the iron oxide film is contained in an amount of from 20 to 100 % by weight.

**4.** A heat generating body, **characterized by** having an exothermic part having a structure in which the wettable heat generating composition compressed body according to claim 1 is interposed between a substrate and a covering material and at least the periphery of the wettable heat generating composition compressed body is sealed.

**5.** The heat generating body according to claim 4, **characterized in that** the exothermic part is an exothermic part in which a plural number of sectional exothermic parts are provided at intervals; that the sectional exothermic parts contain the wettable heat generating composition compressed body; that the wettable heat generating composition compressed body has a height of from 0.1 to 10 mm and a volume of from 0.01 to 30 cm$^3$; and that a ratio of the capacity of the sectional exothermic parts to the voleme of the wettable heat generating composition compressed body is from 0.6 to 1.0.

**6.** The heat generating body according to claim 4, **characterized in that** in the heat generating body provided with two or more of the sectional exothermic parts, the air-permeable surface is covered by an air permeability adjusting material.

**7.** The heat generating body according to claim 4, **characterized in that** an outer periphery of the sealed wettable heat generating composition compressed body is collapsed by an outer pressure.

**8.** The heat generating body according to claim 4, **characterized in that** at least a part of one of the exposed surfaces of the heat generating body has a fixing measure.

**9.** A process for producing a wettable heat generating composition compressed body resulting from compression of a moldable heat generating composition capable of causing an exothermic reaction upon contact with air, which is **characterized by**:

1) using, as a heat generating composition, a moldable heat generating composition prepared by subjecting a reaction mixture containing, as essential components, an iron powder, a carbon component, a reaction accelerator, a crosslinking type water absorptive polymer and water but not containing a flocculant aid, a flocculant, an agglomeration aid, a dry binder, a dry binding agent, a dry binding material, a sticky raw material, a thickener, an excipient, an alcohol, a crosslinking agent and a plasticizer and having a water content of from 1 to 20 % by weight and a water mobility value of less than 0.01 to a contact treatment with an oxidizing gas under circumstances at 0 °C or higher, regulating a temperature rise of the reaction mixture at 1 °C or higher within 10 minutes, and adjusting the water content so as to contain surplus water having a water mobility value of from 0.01 to 20, and

2) filling the moldable heat generating composition in a mold cavity of a mold cavity-provided die to a depth substantially the same as the depth of the mold cavity and compressing to a thickness of from 50 to 99.5 % against the depth of the mold cavity.

## FIG. 1

## FIG. 2

# FIG. 3

# FIG. 4

# *FIG.5*

# *FIG.6*

<table>
<tr><td colspan="2" align="center">**INTERNATIONAL SEARCH REPORT**</td><td>International application No.<br>PCT/JP2005/013002</td></tr>
</table>

| A. CLASSIFICATION OF SUBJECT MATTER |
|---|
| Int.Cl$^7$ A61F7/08, C09K5/16 |

According to International Patent Classification (IPC) or to both national classification and IPC

| B. FIELDS SEARCHED |
|---|

Minimum documentation searched (classification system followed by classification symbols)
Int.Cl$^7$ A61F7/08, C09K5/16

Documentation searched other than minimum documentation to the extent that such documents are included in the fields searched

| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)

| C. DOCUMENTS CONSIDERED TO BE RELEVANT | | |
|---|---|---|
| Category* | Citation of document, with indication, where appropriate, of the relevant passages | Relevant to claim No. |
| A | JP 2002-155273 A  (Kaoru USUI),<br>28 May, 2002 (28.05.02),<br>Full text; all drawings<br>(Family: none) | 1-9 |
| A | JP 7-265347 A  (Yukio KOMATSU),<br>17 October, 1995 (17.10.95),<br>Full text; all drawings<br>(Family: none) | 1-9 |
| A | JP 2003-102761 A  (Kao Corp.),<br>08 April, 2003 (08.04.03),<br>Full text; all drawings<br>& US 2005/0000827 A1     & EP 1437111 A1<br>& WO 2003/028597 A1     & CN 1561187 A | 1-9 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>29 August, 2005 (29.08.05) | Date of mailing of the international search report<br>13 September, 2005 (13.09.05) |
|---|---|
| Name and mailing address of the ISA/<br>Japanese Patent Office | Authorized officer |
| Facsimile No. | Telephone No. |

Form PCT/ISA/210 (second sheet) (January 2004)

**INTERNATIONAL SEARCH REPORT**

International application No.

PCT/JP2005/013002

C (Continuation).   DOCUMENTS CONSIDERED TO BE RELEVANT

| Category* | Citation of document, with indication, where appropriate, of the relevant passages | Relevant to claim No. |
|---|---|---|
| A | JP 1-201253 A  (Japan Pionics Co., Ltd.),<br>14 August, 1989 (14.08.89),<br>Full text; all drawings<br>(Family: none) | 1-9 |
| A | JP 57-200317 A  (Teijin Ltd.),<br>08 December, 1982 (08.12.82),<br>Full text<br>(Family: none) | 1-9 |
| A | JP 57-166156 A  (Dainihon Jochugiku Co., Ltd.),<br>13 October, 1982 (13.10.82),<br>Full text<br>(Family: none) | 1-9 |
| A | JP 56-28266 A  (Matsushita Electric Industrial Co., Ltd.),<br>19 March, 1981 (19.03.81),<br>Full text; all drawings<br>(Family: none) | 1-9 |
| A | JP 2003-509120 A  (The Procter & Gamble Co.),<br>11 March, 2003 (11.03.03),<br>Full text; all drawings<br>& US 6336935 B1        & EP 1212020 A1<br>& WO 2001/019302 A1      & CN 1373649 A | 1-9 |

Form PCT/ISA/210 (continuation of second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 59189183 A
- JP 11508314 T
- WO 0013626 A
- JP 11508786 T
- JP 11512954 T
- JP 2002514104 T
- JP 2003509120 T
- JP 20015075930 T
- JP 2002200108 A
- JP 10265373 A
- JP 9087173 A
- JP 6145050 A
- JP 6199660 A
- JP 10279466 A
- JP 10182408 A
- JP 2001507593 T
- JP 3161605 B
- JP 4293989 A
- JP 6343658 A
- JP 7194641 A